(19) Europäisches Patentamt
European Patent Office
Office européen des brevets

(11) Veröffentlichungsnummer: **0 094 562**
**B1**

(12) # EUROPÄISCHE PATENTSCHRIFT

(45) Veröffentlichungstag der Patentschrift: **05.12.84**

(21) Anmeldenummer: **83104410.2**

(22) Anmeldetag: **05.05.83**

(51) Int. Cl.³: **C 07 C 87/30,** C 07 C 87/46, C 07 C 85/00, C 07 D 295/04, C 07 D 295/02, C 07 C 93/06, C 07 D 265/30, A 01 N 33/12, A 01 N 43/60

(54) **Diaminderivate, Verfahren zu ihrer Herstellung und ihre Verwendung als Fungizide.**

(30) Priorität: **15.05.82 DE 3218394**

(43) Veröffentlichungstag der Anmeldung: **23.11.83 Patentblatt 83/47**

(45) Bekanntmachung des Hinweises auf die Patenterteilung: **05.12.84 Patentblatt 84/49**

(84) Benannte Vertragsstaaten: **AT BE CH DE FR GB IT LI NL SE**

(56) Entgegenhaltungen:
**DE - A - 1 493 745**
**DE - B - 1 126 880**
**DE - C - 836 937**
**GB - A - 1 109 502**

(73) Patentinhaber: **BASF Aktiengesellschaft, Carl-Bosch-Strasse 38, D-6700 Ludwigshafen (DE)**

(72) Erfinder: **Rentzea, Costin, Dr., Neuenheimer Landstrasse 72, D-6900 Heidelberg (DE)**
Erfinder: **Buschmann, Ernst, Dr., Georg-Ludwig-Krebs-Strasse 10, D-6700 Ludwigshafen (DE)**
Erfinder: **Meyer, Norbert, Dr., Stahlbuehlring 155a, D-6802 Ladenburg (DE)**
Erfinder: **Ammermann, Eberhard, Dr., Sachsenstrasse 3, D-6700 Ludwigshafen (DE)**
Erfinder: **Pommer, Ernst-Heinrich, Dr., Berliner Platz 7, D-6703 Limburgerhof (DE)**

EP 0 094 562 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Diaminderivate, Verfahren zu ihrer Herstellung und fungizide Mittel, die diese Verbindungen als Wirkstoffe enthalten.

Es ist bekannt, N-Trichlormethylthiotetrahydrophthalimid als Fungizid zu verwenden („Chemical Week", 21. Juni 1972, S. 46).

Es wurde nun gefunden, dass Diaminderivate der Formel:

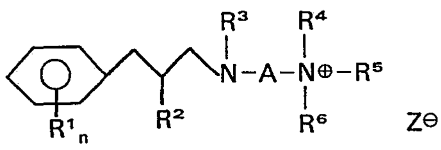 (I)

in der

$R^1$ Alkyl, halogensubstituiertes Alkyl, ggf. substituiertes Aryl oder Aralkyl, Cycloalkyl, Bicycloalkyl, Alkoxy, Acyl oder Halogen,

n 0, 1, 2 oder 3,

$R^2$ Alkyl, Alkenyl oder Alkoxy,

A eine ggf. durch Alkyl, Halogenalkyl, Alkoxy oder Halogen substituierte $C_2$-$C_4$-Kette oder ein ggf. durch Alkyl, Halogenalkyl oder Alkoxy substituiertes $C_5$-$C_7$-Cycloalkylen bedeutet,

$R^3$, $R^4$ und $R^5$ jeweils unabhängig voneinander Alkyl, halogensubstituiertes Alkyl, Alkenyl, Alkinyl oder ggf. durch Halogen, Alkyl, Alkoxy, Trifluormethyl, Cyan oder Nitro substituiertes Benzyl bedeuten, oder $R^3$ mit $R^4$ zusammen mit A und mit den beiden Stickstoffatomen, die an A gebunden sind, einen ggf. durch Alkyl substituierten Piperazin- oder Perhydrodiazepinring bilden oder $R^4$ zusammen mit $R^5$ und dem Stickstoffatom, dessen Substituenten sie sind, einen ggf. durch Alkyl substituierten 5-, 6- oder 7gliedrigen heterocyclischen Ring mit 1 bis 2 Heteroatomen bilden,

$R^6$ Alkyl, Alkenyl, Alkinyl oder ggf. substituiertes Aralkyl oder Aryloxyalkyl bedeutet, und

Z das Anion einer beliebigen, nicht phytotoxischen Säure bedeutet,

eine gute Wirkung gegen Pilze und Bakterien haben.

$R^1$ bedeutet beispielsweise Wasserstoff, Fluor, Chlor, Brom, $C_1$-$C_8$-Alkyl, z.B. Methyl, Ethyl, n-Propyl, Isopropyl, n-Butyl, Isobutyl, tert.-Butyl, n-Pentyl, tert.-Amyl, 1,1-Dimethylbutyl, 1,1-Dimethylpentyl, 1,1-Dimethylhexyl, 1,1-Diethylpropyl, 1,1,2-Trimethylpropyl, Halogen-$C_1$-$C_4$-Alkyl, z.B. 2-Chlor-1,1-dimethylethyl, Trifluormethyl, Cyclohexyl, Phenyl, Halogenphenyl, z.B. 4-Chlorphenyl, Benzyl, Halogenbenzyl, z.B. 4-Chlorbenzyl, Phenylethyl, $C_1$-$C_2$-Alkoxy, z.B. Methoxy, Ethoxy, $C_1$-$C_2$-Alkylcarboxyl, z.B. Acetyl, Propionyl oder Benzoyl.

$R^2$ bedeutet beispielsweise Methyl, Ethyl, Propyl, Butyl, Allyl, Crotyl.

A bedeutet beispielsweise $-(CH_2)_2-$, $-(CH_2)_3-$, $-\overset{\overset{CH_3}{|}}{C}H-CH_2-$, oder $-\overset{\overset{CH_3}{|}}{C}H-CH_2CH_2-$.

In einer weiteren Gruppe bevorzugter Verbindungen bedeutet A die Reste 1,2-, 1,3-, 1,4-Cyclohexylen ( 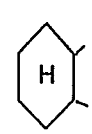 , 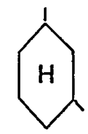 und 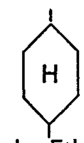 ).

$R^3$ bedeutet beispielsweise Methyl oder Ethyl.

$R^4$ und $R^5$ bedeuten beispielsweise Methyl, Ethyl, Propyl, Allyl, Butenyl, Propargyl, Benzyl, Halogenbenzyl, z.B. 4-Fluorbenzyl, 4-Chlorbenzyl, 3-Chlorbenzyl, 4-Brombenzyl, 3,4-Dichlorbenzyl, 2,4-Dichlorbenzyl, 3-Trifluormethylbenzyl, 4-Trifluormethylbenzyl, 4-Methylbenzyl, 4-(tert.-Butyl)benzyl, 4-Methoxybenzyl, 4-Cyanbenzyl oder 4-Nitrobenzyl.

Bevorzugt werden Verbindungen der Formel I, in der $R^3$ mit $R^4$ zusammen mit A und mit den beiden Stickstoffatomen einen Piperazin- oder einen Perhydrodiazepinring bilden.

Bevorzugt ist ferner eine weitere Gruppe von Verbindungen der Formel I, in der $R^4$ mit $R^5$ zusammen mit N einen ggf. durch Methyl substituierten Pyrrolidin-, Piperidin- oder Morpholinring bilden.

$R^6$ bedeutet beispielsweise Methyl, Ethyl, Propyl, Butyl, Isobutyl, Pentyl, Allyl, 2-Buten-1-yl, 4-Chlor-2-buten-1-yl, Propargyl, 1- oder 2-Naphthylmethyl, Benzyl, substituiertes Benzyl wie 2-Fluorbenzyl, 4-Fluorbenzyl, 2-Chlorbenzyl, 3-Chlorbenzyl, 3-Brombenzyl, 4-Brombenzyl, 3-Trifluormethylbenzyl, 4-Trifluormethylbenzyl, 3-Methylbenzyl, 4-Methylbenzyl, 4-tert.-Butylbenzyl, 4-Cyanbenzyl, 4-Nitrobenzyl, 2,4-Dichlorbenzyl, 3,4-Dichlorbenzyl, 2,4-Dimethylbenzyl, 4-Methoxybenzyl und 2,3,6-Trichlorbenzyl oder 1-(4-tert.-Butylphenyl)-2-methylprop-3-yl oder ferner 1-Phenoxyethyl-2, 1-Phenoxypropyl-3, 1-(3-Chlorphenoxy)ethyl-2, 1-(4-Chlorphenoxy)ethyl-2, 1-(4-Methylphenoxy)-ethyl-2, 1-(4-Methoxyphenoxy)ethyl-2, 1-(4-Nitrophenoxy)propyl-3, 1-(4-Trifluormethylphenoxy)ethyl-2 und 1-Phenoxybutyl-4; Z bedeutet beispielsweise Methylsulfonat, p-Dodecylbenzolsulfonat, Sulfat, Methosulfat, Nitrat, Phosphat, Iodid und insbesondere Chlorid oder Bromid.

Die neuen Diaminderivate der Formel I besitzen ein chirales, an den Liganden $R^2$ gebundenes Kohlenstoffatom und, je nach der Beschaffenheit von $R^1$, $R^3$, $R^4$, $R^5$, $R^6$ und A, noch weitere Chiralitätszentren in diesen Resten. Nach üblichen Methoden können die optisch reinen Enantiomeren bzw. die Diastereomeren erhalten werden. Die vorliegende Erfindung erfasst auch diese Verbindungen in reiner Form oder als Mischungen. Als Fungizide sind sowohl die reinen Enantiomeren bzw. die einheitlichen Diastereomeren als auch die bei der Synthese anfallenden Mischungen wirksam.

Man erhält die Diaminderivate der Formel I beispielsweise durch Umsetzung von Verbindungen der Formel:

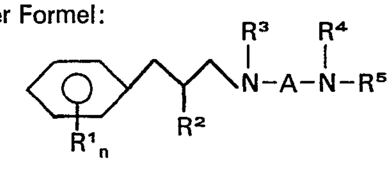 (II)

in der A, $R^1$, $R^2$, $R^3$, $R^4$, $R^5$ und n die oben angegebenen Bedeutungen haben, mit einer Verbindung $R^6Z$, in der $R^6$ und Z die oben angegebenen Bedeutungen haben.

Die Umsetzungen werden ggf. in Gegenwart eines Lösungs- oder Verdünnungsmittels bei einer Temperatur im Bereich zwischen 20 und 150, vorzugsweise zwischen 30 und 140° C, durchgeführt. Der Ausgangsstoff der Formel II wird zweckmässig mit der molaren Menge der Verbindung $R^6Z$ umgesetzt.

Als bevorzugte, gegenüber den Reaktionsteilnehmern inerte Lösungs- oder Verdünnungsmittel können beispielsweise aliphatische oder aromatische, ggf. halogenierte Kohlenwasserstoffe wie Pentan, Cyclohexan, Benzol, Toluol, Xylole, Chlorbenzol; aliphatische Ketone wie Aceton, Methylethylketon, Diethylketon oder Cyclopentanon; Ether wie Diethylether, Dimethoxyethan, Methyltert.-butylether, Tetrahydrofuran oder Dioxan; Ester wie Essigsäureethylester; Nitrile wie Acetonitril; Amide wie Dimethylformamid, Dimethylacetamid und N-Methylpyrrolidon, oder Gemische dieser Lösungsmittel verwendet werden.

Die Herstellung der Ausgangsverbindungen der Formel II nach an sich bekannten Verfahren wird zusammen mit der Synthese der neuen Verbindungen der Formel I beschrieben.

Als Quaternierungsmittel der Formel $R^6Z$ kommen ausser den Alkyl-, Alkenyl-, Alkinyl-, Benzyl- bzw. Aralkylhalogeniden z.B. in Betracht: Dimethylsulfat, Diethylsulfat, Sulfonsäureester der Formel $R-SO_3-R^6$, wobei $R^6$ $C_1$-$C_7$-Alkyl oder durch Halogen oder Alkyl substituiertes Phenyl oder Aralkyl bedeutet kann.

Die folgenden Beispiele erläutern die Herstellung der Verbindungen der Formel I:

*Beispiel 1:*

a) 274 g trans-1,2-Diaminocyclohexan und 134,4 g 3-(4-tert.-Butylphenyl)-2-methylpropylchlorid (DE-OS Nr. 2752096) wurden 10 h bei 140° C gerührt, auf +10° C (Eisbad) abgekühlt und langsam mit 200 ml 50%iger (Gew.-%) Natronlauge versetzt. Nach Zugabe von 200 ml Ether wurde die organische Phase abgetrennt, über Kaliumhydroxid getrocknet und destilliert. Man erhielt 151 g N-(3-(4-tert.-Butylphenyl)-2-methylpropyl)-trans-1,2-diaminocyclohexan vom Sdp. 175 bis 180° C/0,3 mbar; Ausbeute 83,3% d.Th.

Aus einem Gemisch von 70% trans- und 30% cis-1,2-Diaminocyclohexan (274 g) erhielt man nach dem obigen Verfahren 154,1 g N-(3-(4-tert.-Butylphenyl)-2-methylpropyl)-1,2-diaminocyclohexan als cis/trans-Gemisch vom Sdp. 175 bis 185° C/0,3 mbar; Ausbeute 85% d.Th.

b) Eine Mischung aus 260 ml 98%iger Ameisensäure und 400 ml 40%iger wässerigen Formaldehydlösung wurde zwischen 50 und 80° C mit 151 g N-(3-(4-tert.-Butylphenyl)-2-methylpropyl)-1,2-diaminocyclohexan (trans-Verbindung) innerhalb 30 min tropfenweise versetzt. Nach zehnstündigem Rühren bei 90 bis 95° C wurde das Gemisch auf +10° C abgekühlt, mit 234 ml 36%iger Salzsäure vorsichtig versetzt und anschliessend im Vakuum eingeengt. Der Rückstand wurde mit 940 ml 30%iger Natronlauge gerührt und dreimal mit je 150 ml Ether ausgeschüttelt. Der Etherextrakt wurde über Kaliumhydroxid getrocknet und destilliert. Man erhielt 132 g N′, N″, N″-Trimethyl-N′-(3-(4-tert.-butylphenyl)-2-methylpropyl)cyclohexan-1,2-diamin vom Sdp. 180 bis 190° C/0,3 mbar und $n_D^{25} = 1{,}5097$; Ausbeute 76,5% d.Th.

c) Zu einer Lösung von 10,5 g N′, N″, N″-Trimethyl-N′-(3-(4-tert.-butylphenyl)-2-methylpropyl)cyclohexan-1,2-diamin in 10 ml Dioxan und 45 ml Acetonitril wurden 9,2 g 4-tert.-Butylbenzylchlorid zugegeben und das Gemisch 16 h bei 80° C gerührt. Nach Einengen im Vakuum wurde der Rückstand dreimal mit je 20 ml Ether gewaschen und im Vakuum bei 80° C und 0,2 mbar 3 h getrocknet. Man erhielt 11 g 1-{N-[3-(4-tert.-Butylphenyl)-2-methylpropyl]-N-methyl}aminocyclohexan-2-[N,N-dimethyl-N-(4-tert.-butylbenzyl)]ammoniumchlorid als hellgelbes Harz; Ausbeute 68,7% d.Th. (Verbindung 1.)

IR-Spektrum (Film): 3022, 2964, 2866, 1512, 1476, 1463, 1364, 1270, 1110, 1030, 843 und 563 $cm^{-1}$.

*Beispiel 2:*

a) 57 g trans-1,2-Diaminocyclohexan, 208 g 3-(4-tert.-Butylphenyl)-2-methylpropanal und 3 ml Essigsäure wurden in 300 ml Toluol für 5 h unter Rückfluss erhitzt, bis 18 ml Wasser mit Toluol azeotrop abdestilliert waren. Dann wurde das Toluol unter vermindertem Druck abdestilliert, und der Rückstand wurde direkt weiter umgesetzt. Die Ausbeute an Schiffscher Base

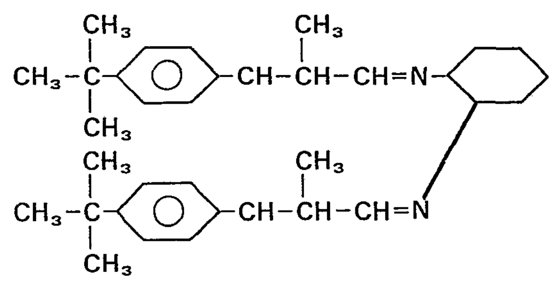

betrug 223 g, entsprechend 91,7% d.Th.

b) 200 g der Schiffschen Base wurden in 500 ml Methanol gelöst und bei 0° C portionsweise mit 38 g Natriumborhydrid versetzt. Nach zwölfstündigem Rühren bei 20° C wurde das Methanol abdestilliert, der Rückstand wurde in 500 ml Methylenchlorid gelöst und 1 h mit 300 ml 25%iger Kalilauge gerührt. Dann wurde die organische Phase abgetrennt, zweimal mit je 200 ml Wasser gewaschen und über Natriumsulfat getrocknet. Das Methylenchlorid wurde im Vakuum abdestilliert. Der Rückstand war N′,N″-Bis[3-(4-tert.-butylphenyl)-2-methylpropyl]-1,2-diaminocyclohexan. Die Ausbeute betrug 195 g hellbraunes Harz, entsprechend 97,5% d.Th.

c) Einer Mischung aus 134 ml 40%iger wässeriger Formaldehydlösung und 86,6 ml 98%iger Ameisensäure wurden 200 g N′,N″-Bis-[3-(4-tert.-butylphenyl)-2-methylpropyl]-1,2-diaminocyclohexan zwischen 50 und 80° C portionsweise zugegeben. Nach zehnstündigem Rühren bei 95° C wurde das Gemisch auf +10° C abgekühlt, mit 234 ml 36%iger Salzsäure vorsichtig versetzt und anschliessend unter vermindertem Druck eingeengt. Der Rückstand wurde mit 940 ml 30%iger Natronlauge gerührt und viermal mit je 150 ml Ether ausgeschüttelt. Der Etherextrakt wurde zweimal mit je 100 ml Wasser gewaschen, über Kaliumhydroxid getrocknet und im Vakuum bei 100° C und 0,2 mbar 3 h getrocknet. Die Ausbeute an N',N''-Dimethyl-N',N''-bis-[-3-(4-tert.-butylphenyl)-2-methylpropyl]-1,2-diaminocyclohexan betrug 185,4 g hellbraunes Harz, entsprechend 87,7% d.Th.

d) Zu einer Lösung von 10,9 g N',N''-Bis-[3-(4-tert.-butylphenyl)-2-methylpropyl]-2-methyl-propyl-1,2-diaminocyclohexan in 20 ml Dioxan und 30 ml Acetonitril wurden 7,2 g Benzylbromid zugegeben und das Gemisch 3 d bei 70° C gerührt. Nach Einengen im Vakuum wurde der Rückstand dreimal mit Petrolether gewaschen und zum Schluss bei 100° C und 0,2 mbar 4 h getrocknet. Man erhielt 6,6 g 1-{N-Methyl-N-[3-(4-tert.-butylphenyl)-2-methylpropyl]}aminocyclohexan-2-{-N-methyl-N-benzyl-N-[3-(4-tert.-butylphenyl)-2-methylpropyl]}ammoniumbromid als hellbraunes Harz; Ausbeute 45,6% d.Th. (Verbindung 2.)

IR-Spektrum (Film): 3030, 2962, 2866, 1515, 1460, 1393, 1364, 1269, 1204, 1109, 853 und 706 $cm^{-1}$.

*Beispiel 3:*

a) 533 g 3-(4-tert.-Butylphenyl)-2-methylpropylchlorid und 174 g 4-Methylpiperazin wurden 7 h bei 140° C gerührt, auf +10° C (Eisbad) abgekühlt und langsam mit 400 ml 50%iger Natronlauge versetzt. Nach Zugabe von 500 ml Ether wurde die organische Phase abgetrennt, über Kaliumhydroxid getrocknet und destilliert. Man erhielt 640 g 1-Methyl-4-[3-(4-tert.-butylphenyl)-2-methylpropyl]piperazin vom Sdp. 135 bis 140° C/0,3 mbar.

b) Eine Lösung von 14,4 g 1-Methyl-4-[3-(4-tert.-butylphenyl)-2-methylpropyl]piperazin und 10,3 g Benzylbromid in 200 ml Essigester wurde 6 h zum Rückfluss erwärmt. Das Gemisch wurde anschliessend auf 80 ml eingeengt, auf +5° C abgekühlt und das ausgefallene Produkt abgesaugt, mit Ether gewaschen und getrocknet. Man erhielt 18,4 g 1-Methyl-1-benzyl-4-[3-(4-tert.-butylphenyl)-2-methylpropyl]piperaziniumbromid als farblose Kristalle vom Smp. 216° C. (Verbindung 3.)

Entsprechend wurden die folgenden Verbindungen hergestellt.

*(Tabellen →)*

Die Wirkstoffe zeigen eine starke Wirksamkeit gegen Mikroorganismen. Sie dienen insbesondere zur Verhütung und Heilung von Pflanzenkrankheiten, die durch Pilze verursacht werden, wie z.B. *Botrytis cinerea* an Reben und Erdbeeren, *Monilia fructigena* an Äpfeln, *Phytophthora infestans* an Kartoffeln und Tomaten, *Plasmopara viticola* an Reben und *Alternaria solani* an Tomaten.

Die fungiziden bzw. bakteriziden Mittel enthalten 0,1 bis 95% (Gew.-%) Wirkstoff, vorzugsweise 0,5 bis 90%. Die Aufwandmengen liegen je nach Art des gewünschten Effektes zwischen 0,1 und 5 kg Wirkstoff je Hektar.

Auch für technische Zwecke, zum Schutz technischer Materialien, z.B. als Holzschutzmittel, sind die erfindungsgemässen Imidazoliumsalze geeignet. Über die fungizide Wirkung hinaus wurde auch eine bakterizide Wirksamkeit der Verbindungen festgestellt, so dass sie auch als solche im Pflanzenschutz und als technische Mikrozide in Betracht kommen. Beispielsweise können folgende Mikroorganismen damit bekämpft werden.

*Staphylococcus aureus, Escherichia coli, Klebsiella pneumoniae, Citrobacter freundii, Proteus vulgaris, Pseudomonas aeruginosa, Pseudomonas fluorescens, Xanthomonas vesicatoria, Xanthomonas malvaccarum, Erwinia carotovora, Erwinia amylovora, Desulfovibrio desulfuricans, Streptoverticillium rubrireticuli, Aspergillus niger, Aspergillus versicolor, Penicillium funiculosum, Paecilomyces variotii, Trichoderma viride, Chaetomium globosum, Candida albicans, Geotrichum candidans, Monilia sitophila, Scenedesmus quadricauda, Chlorella vulgaris, Nostoc muscorum.*

Die üblichen Anwendungskonzentrationen betragen 0,01 bis 1% Wirkstoff, bezogen auf das Gewicht des zu schützenden Materials; beim Einsatz zur Wasserbehandlung bei der Erdölförderung, in Schwimmbädern, Rückkühlwerken, Luftbefeuchtungsanlagen, Blumenfrischhaltemitteln oder in der Papierindustrie sind Wirkstoffmengen von 5 bis 100 ppm ausreichend, um eine Mikroorganismenentwicklung zu unterdrücken. Gebrauchsfertige Desinfektionsmittellösungen enthalten 0,2 bis 5% Wirkstoff.

Die Wirkstoffe können auch zusammen mit anderen Wirkstoffen, z.B. Herbiziden, Insektiziden, Wachstumsregulatoren und anderen Fungiziden, oder auch mit Düngemitteln vermischt und ausgebracht werden. In vielen Fällen erhält man bei der Mischung mit Fungiziden auch eine Vergrösserung des fungiziden Wirkungsspektrums; bei einer Anzahl dieser Fungizidmischungen treten auch synergistische Effekte auf, d.h. die fungizide Wirksamkeit des Kombinationsproduktes ist grösser als die der addierten Wirksamkeiten der Einzelkomponenten.

Fungizide, die mit den neuen Verbindungen kombiniert werden können, sind beispielsweise:
Schwefel,
Dithiocarbamate und deren Derivate, wie
Ferridimethyldithiocarbamat,
Zinkdimethyldithiocarbamat,
Manganzinkethylendiaminbisdithiocarbamat und
Zinkethylenbisdithiocarbamat,
Tetramethylthiuramdisulfide,
Ammoniakklompex von Zink-(N,N-ethylenbisdithiocarbamat) und
N,N'-Polyethylenbis(thiocarbamoyl)disulfid,
Zink-(N,N'-propylenbisdithiocarbamat),

→

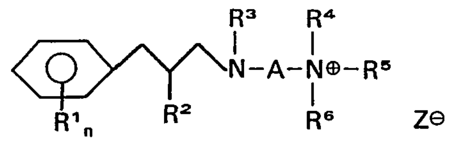

*Tabelle*

| Nr. | $R^1$ | $R^2$ | $R^3$ | A | $R^4$ | $R^5$ | $R^6$ | Z | Phys. Konstante oder IR-Spektrum ($cm^{-1}$) (Film) |
|---|---|---|---|---|---|---|---|---|---|
| 4 | 4-$C(CH_3)_3$ | $-CH_3$ | $-CH_3$ | $-(CH_2)_2$ | $-CH_3$ | $-CH_3$ | 2,4-$Cl_2C_6H_3-CH_2-$ | Cl | 3002, 2963, 1589, 1475, 1384, 1105, 1055, 869, 849, 786 |
| 5 | 4-$C(CH_3)_3$ | $-CH_3$ | $-CH_3$ | $-(CH_2)_2$ | $-CH_3$ | $-CH_3$ | 3,4-$Cl_2C_6H_3-CH_2-$ | Cl | 2962, 1471, 1219, 1136, 1035, 826, 672 |
| 6 | 4-$C(CH_3)_3$ | $-CH_3$ | $-CH_3$ | $-(CH_2)_3$ | $-CH_3$ | $-CH_3$ | 4-(tert.-$C_4H_9$)$C_6H_4-CH_2-$ | Cl | Smp. 201-207°C |
| 7 | 4-$C(CH_3)_3$ | $-CH_3$ | $-CH_3$ | $-(CH_2)_3$ | $-CH_3$ | $-CH_3$ | 4-$ClC_6H_4-CH_2-$ | Cl | 2963, 1599, 1493, 1414, 1270, 1216, 1093, 1017, 852, 818 |
| 8 | 4-$C(CH_3)_3$ | $-CH_3$ | $-CH_3$ | $-(CH_2)_3$ | $-CH_3$ | $-CH_3$ | 2,4-$Cl_2C_6H_3-CH_2-$ | Cl | 3010, 2963, 2869, 1589, 1475, 1384, 1107, 868, 827 |
| 9 | 4-$C(CH_3)_3$ | $-CH_3$ | $-CH_3$ | 1,2-Cyclohexylen | $-CH_3$ | $-CH_3$ | Allyl | Br | Harz |
| 10 | 4-$C(CH_3)_3$ | $-CH_3$ | $-CH_3$ | 1,2-Cyclohexylen | $-CH_3$ | $-CH_3$ | Buten-2-yl-1 | Br | Harz |
| 11 | 4-$C(CH_3)_3$ | $-CH_3$ | $-CH_3$ | 1,2-Cyclohexylen | $-CH_3$ | $-CH_3$ | Propargyl | Br | Harz |
| 12 | 4-$C(CH_3)_3$ | $-CH_3$ | $-CH_3$ | 1,2-Cyclohexylen | $-CH_3$ | $-CH_3$ | $Cl-CH_2-CH=CH-CH_2$ | Cl | Harz |
| 13 | 4-$C(CH_3)_3$ | $-CH_3$ | $-CH_3$ | 1,2-Cyclohexylen | $-CH_3$ | $-CH_3$ | $C_6H_5-CH_2-$ | Br | 2963, 2864, 1510, 1375, 1220, 1027, 850, 735, 578 |
| 14 | 4-$C(CH_3)_3$ | $-CH_3$ | $-CH_3$ | 1,2-Cyclohexylen | $-CH_3$ | $-CH_3$ | 4-$ClC_6H_4-CH_2$ | Cl | 3014, 2963, 2862, 1512, 1490, 1377, 1090, 1030, 1015, 855 |
| 15 | 4-$C(CH_3)_3$ | $-CH_3$ | $-CH_3$ | 1,2-Cyclohexylen | $-CH_3$ | $-CH_3$ | 2,4-$Cl_2C_6H_3-CH_2-$ | Cl | 3020, 2964, 2865, 1588, 1510, 1475, 1382, 1102, 864, 842 |
| 16 | 4-$C(CH_3)_3$ | $-CH_3$ | $-CH_3$ | 1,2-Cyclohexylen | $-CH_3$ | $-CH_3$ | 1-$C_{10}H_7-CH_2-$ | Cl | 3022, 2958, 2868, 1514, 1461, 1362, 1270, 1110, 1030, 810, 784 |
| 17 | 4-$C(CH_3)_3$ | $-CH_3$ | $-CH_3$ | 1,2-Cyclohexylen | $-CH_3$ | $-CH_3$ | $C_6H_5-CH=CH-CH_2-$ | Br | 2954, 2866, 1450, 1365, 1270, 1031, 986, 850, 758, 695, 575 |

| Nr. | $R^1$ | $R^2$ | $R^3$ | A | $R^4$ | $R^5$ | $R^6$ | Z | Phys. Konstante oder IR-Spektrum ($cm^{-1}$) (Film) |
|---|---|---|---|---|---|---|---|---|---|
| 18 | 4-$C(CH_3)_3$ | $-CH_3$ | $-CH_3$ | 1,2-Cyclo-hexylen | $-CH_3$ | $-CH_3$ | 4-$FC_6H_4-CH_2-$ | Cl | 2960, 2867, 1607, 1513, 1476, 1462, 1229, 1030, 865, 845 |
| 19 | 4-$C(CH_3)_3$ | $-CH_3$ | $-CH_3$ | 1,2-Cyclo-hexylen | $-CH_3$ | $-CH_3$ | 4-$(NO_2)C_6H_4-CH_2-$ | Br | 2961, 2865, 1607, 1525, 1476, 1463, 1347, 1109, 842, 738 |
| 20 | 4-$C(CH_3)_3$ | $-CH_3$ | $-CH_3$ | 1,2-Cyclo-hexylen | $-CH_3$ | $-CH_3$ | 2,4-$(CH_3)_2C_6H_3-CH_2-$ | Cl | 3020, 2960, 2920, 2854, 1507, 1457, 1437, 1385, 1270, 1110, 830 |
| 21 | 4-$C(CH_3)_3$ | $-CH_3$ | $-CH_3$ | 1,2-Cyclo-hexylen | $-CH_3$ | $-CH_3$ | 4-$BrC_6H_4-CH_2-$ | Br | 2953, 2865, 1488, 1461, 1364, 1072, 1013, 851, 844, 800 |
| 22 | 4-$C(CH_3)_3$ | $-CH_3$ | $-CH_3$ | 1,2-Cyclo-hexylen | $-CH_3$ | $-CH_3$ | 3-$(CF_3)C_6H_4-CH_2-$ | Cl | 2963, 1468, 1331, 1206, 1168, 1126, 1077, 1030, 815, 710 |
| 23 | 4-$C(CH_3)_3$ | $-CH_3$ | $-CH_3$ | 1,2-Cyclo-hexylen | $-CH_3$ | $-CH_3$ | 4-$(CH_3)C_6H_4-CH_2-$ | Cl | 2962, 2865, 1516, 1476, 1462, 1364, 1270, 1110, 1029, 825 |
| 24 | 4-$C(CH_3)_3$ | $-CH_3$ | $-CH_3$ | 1,2-Cyclo-hexylen | $-CH_3$ | $-CH_3$ | $C_6H_5-O-CH_2-CH_2-$ | Br | 2961, 1600, 1498, 1459, 1365, 1239, 1048, 1030, 850, 755 |
| 25 | 4-$C(CH_3)_3$ | $-CH_3$ | $-CH_3$ | 1,2-Cyclo-hexylen | $-CH_3$ | $-CH_3$ | 3-$ClC_6H_4-O-(CH_2)_2-$ | Br | 2959, 1595, 1476, 1284, 1230, 1047, 1030, 975, 850, 775 |
| 26 | 4-$C(CH_3)_2C_2H_5$ | $-CH_3$ | $-CH_3$ | 1,2-Cyclo-hexylen | $-CH_3$ | $-CH_3$ | $C_6H_5-CH_2$ | Br | 2963, 2932, 2864, 1511, 1458, 1377, 1220, 1029, 851, 705 |
| 27 | 4-$C(CH_3)_2C_2H_5$ | $-CH_3$ | $-CH_3$ | 1,2-Cyclo-hexylen | $-CH_3$ | $-CH_3$ | 4-(tert.-$C_4H_9)C_6H_4-CH_2-$ | Cl | 2964, 2929, 2866, 1513, 1462, 1363, 1270, 1110, 863, 843 |
| 28 | 4-$C(CH_3)_2C_2H_5$ | $-CH_3$ | $-CH_3$ | 1,2-Cyclo-hexylen | $-CH_3$ | $-CH_3$ | 2,4-$Cl_2C_6H_3-CH_2-$ | Cl | 2964, 2866, 1588, 1511, 1475, 1384, 1102, 1053, 1030, 865 |
| 29 | 4-$C(CH_3)_2C_2H_5$ | $-CH_3$ | $-CH_3$ | 1,2-Cyclo-hexylen | $-CH_3$ | $-CH_3$ | 4-$ClC_6H_4-CH_2$ | Cl | 2963, 2864, 1492, 1462, 1377, 1093, 1017, 854, 808, 776 |

| Nr. | $R^1$ | $R^2$ | $R^3$ | A | $R^4$ | $R^5$ | $R^6$ | Z | Phys. Konstante oder IR-Spektrum ($cm^{-1}$) (Film) |
|---|---|---|---|---|---|---|---|---|---|
| 30 | 4-$C(CH_3)_3$ | $-CH_3$ | $-CH_3$ | 1,2-Cyclo-hexylen | $-CH_3$ | n-$C_3H_7-$ | 4-(tert.-$C_4H_9)C_6H_4-CH_2\overset{CH_3}{CH}-CH_2$ | Br | 2963, 2867, 1512, 1462, 1364, 1269, 1110, 1050, 853 |
| 31 | 4-$C(CH_3)_3$ | $-CH_3$ | $-CH_3$ | 1,2-Cyclo-hexylen | $-CH_3$ | $CH_2=CH-CH_2-$ | 4-(tert.-$C_4H_9)C_6H_4-CH_2\overset{CH_3}{CH}-CH_2$ | Br | 2963, 2868, 1512, 1462, 1364, 1268, 1109, 1020, 852, 826 |
| 32 | 4-$C(CH_3)_3$ | $-CH_3$ | $-CH_3$ | 1,2-Cyclo-hexylen | $-CH_3$ | 4-$ClC_6H_4-CH_2-$ | 4-(tert.-$C_4H_9)C_6H_4-CH_2\overset{CH_3}{CH}-CH_2$ | Cl | 2963, 2867, 1512, 1462, 1364, 1269, 1110, 1094, 843, 810 |
| 33 | 4-$C(CH_3)_3$ | $-CH_3$ | $-CH_3$ | 1,2-Cyclo-hexylen | $-CH_3$ | 2,4-$Cl_2C_6H_3-CH_2$ | 4-(tert.-$C_4H_9)C_6H_4-CH_2\overset{CH_3}{CH}-CH_2$ | Cl | 2962, 2867, 1512, 1385, 1364, 1269, 1200, 1109, 1052, 850 |
| 34 | 4-$C(CH_3)_3$ | $-CH_3$ | $-CH_3$ | 1,2-Cyclo-hexylen | $-CH_3$ | 3,4-$Cl_2C_6H_3-CH_2$ | 4-(tert.-$C_4H_9)C_6H_4-CH_2\overset{CH_3}{CH}-CH_2$ | Cl | 2961, 1512, 1473, 1364, 1269, 1109, 1030, 840, 822 |
| 35 | 4-$C(CH_3)_3$ | $-CH_3$ | $-CH_3$ | 1,2-Cyclo-hexylen | $-CH_3$ | 4-(tert.-$C_4H_9)C_6H_4-CH_2-$ | 4-(tert.-$C_4H_9)C_6H_4-CH_2\overset{CH_3}{CH}-CH_2$ | Cl | 2963, 1513, 1462, 1364, 1269, 1123, 1109, 1020, 845 |
| 36 | 4-$C(CH_3)_3$ | $-CH_3$ | $-CH_3$ | 1,4-Cyclo-hexylen | $-CH_3$ | $-CH_3$ | 4-$ClC_6H_4-CH_2-$ | Br | Smp. 160-165°C |
| 37 | 4-$C(CH_3)_3$ | $-CH_3$ | $-CH_3$ | 1,4-Cyclo-hexylen | $-CH_3$ | $-CH_3$ | 2,4-$Cl_2C_6H_3-CH_2-$ | Cl | Smp. 152-155°C |
| 38 | 4-$C(CH_3)_3$ | $-CH_3$ | $-CH_3$ | 1,4-Cyclo-hexylen | $-CH_3$ | $-CH_3$ | Allyl | Br | Smp. 132-136°C |
| 39 | 4-$C(CH_3)_3$ | $-CH_3$ | $-CH_3$ | 1,4-Cyclo-hexylen | $-CH_3$ | $-CH_3$ | 4-$CH_3-C_6H_4-CH_2-$ | Cl | Smp. 161-164°C |
| 40 | 4-$C(CH_3)_3$ | $-CH_3$ | $-CH_3$ | 1,4-Cyclo-hexylen | $-CH_3$ | $-CH_3$ | 4-(tert.-$C_4H_9)C_6H_4-CH_2-$ | Br | Smp. 184-190°C |
| 41 | 4-$C(CH_3)_3$ | $-CH_3$ | $-CH_3$ | 1,4-Cyclo-hexylen | $-CH_3$ | $-CH_3$ | $-CH_3$ | Br | Smp. 232-234°C |
| 42 | 4-$C(CH_3)_3$ | $-CH_3$ | $-CH_3$ | 1,3-Cyclo-hexylen | $-CH_3$ | $-CH_3$ | $C_6H_5-CH_2-$ | Br | Harz |

| Nr. | $R^1$ | $R^2$ | $R^3$ | A | $R^4$ | $R^5$ | $R^6$ | Z | Phys. Konstante oder IR-Spektrum ($cm^{-1}$) (Film) |
|---|---|---|---|---|---|---|---|---|---|
| 43 | $4-C(CH_3)_3$ | $-CH_3$ | $-CH_3$ | 1,3-Cyclo-hexylen | $-CH_3$ | $-CH_3$ | $4-ClC_6H_4-CH_2-$ | Cl | 3018, 2966, 1605, 1598, 1492, 1412, 1215, 1019, 854, 820 |
| 44 | $4-C(CH_3)_3$ | $-CH_3$ | $-CH_3$ | 1,3-Cyclo-hexylen | $-CH_3$ | $-CH_3$ | $2,4-Cl_2C_6H_3-CH_2-$ | Cl | 3012, 2962, 2868, 1587, 1475, 1390, 1105, 868, 825 |
| 45 | $4-C(CH_3)_3$ | $-CH_3$ | $-CH_3$ | 1,3-Cyclo-hexylen | $-CH_3$ | $-CH_3$ | $n-C_5H_{11}$ | J | Harz |
| 46 | $4-C(CH_3)_3$ | $-CH_3$ | $-CH_3$ | $-CH_2-\overset{CH_3}{\overset{\vert}{CH}}-$ | $-CH_3$ | $-CH_3$ | $4-ClC_6H_4-CH_2-$ | Br | Smp. 85-87°C (Zers.) |
| 47 | $4-C(CH_3)_3$ | $-CH_3$ | $-CH_3$ | $-CH_2-\overset{CH_3}{\overset{\vert}{CH}}-$ | $-CH_3$ | $-CH_3$ | $C_6H_5-CH_2-$ | Br | Smp. 97-102°C |
| 48 | $4-C(CH_3)_3$ | $-CH_3$ | $-CH_3$ | $-CH_2-\overset{CH_3}{\overset{\vert}{CH}}-$ | $-CH_3$ | $-CH_3$ | Allyl | Br | Harz |
| 49 | $4-C(CH_3)_3$ | $-CH_3$ | $-CH_3$ | $-CH_2-\overset{CH_3}{\overset{\vert}{CH}}-$ | $-CH_3$ | $-CH_3$ | $-CH_3$ | Br | Smp. 189-192°C |
| 50 | $4-C(CH_3)_3$ | $-CH_3$ | $-CH_3$ | $-CH_2-\overset{CH_3}{\overset{\vert}{CH}}-$ | $-CH_3$ | $-CH_3$ | $4-(CH_3)C_6H_4-CH_2-$ | Cl | Harz |
| 106 | $4-C(CH_3)_3$ | $-CH_3$ | $-C_2H_4-$ | $-C_3H_6-$ | $-C_2H_4-$ | $-CH_3$ | $4-Cl-C_6H_4-CH_2$ | Cl | Smp. 100-102°C |

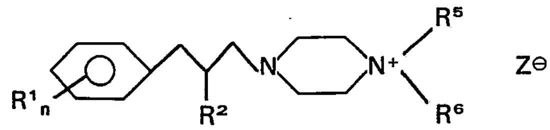

| Nr. | $R^1$ | $R^2$ | $R^5$ | $R^6$ | Z | Fp./°C |
|---|---|---|---|---|---|---|
| 51 | H | $-CH_3$ | $-CH_3$ | $4-ClC_6H_4-CH_2-$ | Cl | 206 |
| 52 | $4-CH_3-CO-$ | $-CH_3$ | $-CH_3$ | $4-ClC_6H_4-CH_2-$ | Cl | 186 |
| 53 | $4-C(CH_3)_3$ | $-CH_3$ | $-CH_3$ | $-CH_3$ | Br | 230-232 |
| 54 | $4-C(CH_3)_3$ | $-CH_3$ | $-CH_3$ | $-CH_3$ | J | 234 (Zers.) |
| 55 | $4-C(CH_3)_3$ | $-CH_3$ | $-CH_3$ | Allyl | Br | 146 |
| 56 | $4-C(CH_3)_3$ | $-CH_3$ | $-CH_3$ | Propargyl | Br | 215 |
| 57 | $4-C(CH_3)_3$ | $-CH_3$ | $-CH_3$ | $4-ClC_6H_4-CH_2-$ | Cl | 216 |
| 58 | $4-C(CH_3)_3$ | $-CH_3$ | $-CH_3$ | $4-ClC_6H_4-CH_2-$ | Br | 218 |
| 59 | $4-C(CH_3)_3$ | $-CH_3$ | $-CH_3$ | $3-ClC_6H_4-CH_2-$ | Cl | 205 |
| 60 | $4-C(CH_3)_3$ | $-CH_3$ | $-CH_3$ | $4-FC_6H_4-CH_2-$ | Cl | 211-212 |
| 61 | $4-C(CH_3)_3$ | $-CH_3$ | $-CH_3$ | $4-(CN)C_6H_4-CH_2-$ | Cl | 223 |
| 62 | $4-C(CH_3)_3$ | $-CH_3$ | $-CH_3$ | $4-(CH_3)C_6H_4-CH_2-$ | Cl | 216 |
| 63 | $4-C(CH_3)_3$ | $-CH_3$ | $-CH_3$ | 4-(tert.-$C_4H_9$)$C_6H_4-CH_2-$ | Cl | 217 |
| 64 | $4-C(CH_3)_3$ | $-CH_3$ | $-CH_3$ | $3,4-Cl_2C_6H_3-CH_2-$ | Cl | 206 |
| 65 | $4-C(CH_3)_3$ | $-CH_3$ | $-CH_3$ | $2,3,6-Cl_3C_6H_2-CH_2-$ | Cl | 91 |
| 66 | $4-C(CH_3)_3$ | $-CH_3$ | Propargyl | $4-ClC_6H_4-CH_2-$ | Br | 162 |
| 67 | $4-C(CH_3)_2C_2H_5$ | $-CH_3$ | $-CH_3$ | $4-ClC_6H_4-CH_2-$ | Cl | 216 |
| 68 | $4-C(CH_3)_2C_3H_7$-n-2-Cl | $-CH_3$ | $-CH_3$ | $C_6H_5-CH_2-$ | Br | 211 |
| 69 | $4-C(CH_3)_2C_3H_7$-n-2-Cl | $-CH_3$ | $-CH_3$ | $4-ClC_6H_4-CH_2-$ | Cl | 192 |
| 70 | $4-C(CH_3)_2C_3H_7$-n-2-Cl | $-CH_3$ | $-CH_3$ | 4-(tert.-$C_4H_9$)$C_6H_4-CH_2-$ | Cl | 204 |
| 71 | $4-C(CH_3)_2C_3H_7$-i | $-CH_3$ | $-CH_3$ | $-CH_3$ | Br | 250 |
| 72 | $4-C(CH_3)_2C_3H_7$-i | $-CH_3$ | $-CH_3$ | Allyl | Br | 135 |
| 73 | $4-C(CH_3)_2C_3H_7$-i | $-CH_3$ | $-CH_3$ | $4-ClC_6H_4-CH_2-$ | Br | 219 |
| 74 | $4-C(CH_3)_2C_3H_7$-i | $-CH_3$ | $-CH_3$ | 4-(tert.-$C_4H_9$)$C_6H_4-CH_2-$ | Br | 198 |
| 75 | $4-C(CH_3)_2C_4H_9$-n | $-CH_3$ | $-CH_3$ | $C_6H_5-CH_2-$ | Cl | 166 |
| 76 | $4-C(CH_3)_2C_4H_9$-n | $-CH_3$ | $-CH_3$ | $4-ClC_6H_4-CH_2-$ | Cl | 194 |
| 77 | $4-C(CH_3)_2C_4H_9$-n | $-CH_3$ | $-CH_3$ | $4-BrC_6H_4-CH_2-$ | Br | 199-201 |
| 78 | $4-C(CH_3)_2C_4H_9$-n | $-CH_3$ | $-CH_3$ | $2,4-Cl_2C_6H_3-CH_2-$ | Cl | 176 |
| 79 | $2,4-Cl_2$ | $-C_3H_7$-n | $-CH_3$ | $-CH_3$ | J | 175 |
| 80 | $2,4-Cl_2$ | $-C_3H_7$-n | $-CH_3$ | Allyl | Br | 193 |
| 81 | $2,4-Cl_2$ | $-C_3H_7$-n | $-CH_3$ | $C_6H_5-CH_2-$ | Br | 166 |
| 82 | $2,3,4-Cl_3$ | $-C_4H_9$-n | $-CH_3$ | $4-ClC_6H_4-CH_2-$ | Cl | 150 |
| 83 | $2,3,4-Cl_3$ | $-C_4H_9$-n | $-CH_3$ | 4-(tert.-$C_4H_9$)$C_6H_4-CH_2-$ | Cl | 163 |
| 99 | $2,6-Cl_2$ | $-CH_3$ | $-CH_3$ | $4-Cl-C_6H_4-CH_2$ | Cl | 214-215 |
| | 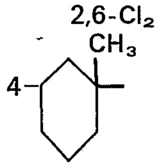 | | | | | |
| 100 | 4-Cyclohexyl | $-CH_3$ | $-CH_3$ | $4-Cl-C_6H_4-CH_2$ | Cl | 117-118 |
| 101 | 4-t-Butyl | $-CH_3$ | $-CH_3$ | 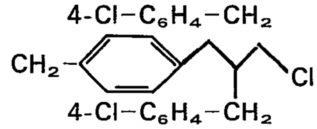 | Cl | 178-180 |
| 102 | 4-t-Butyl | $-CH_3$ | $-C_2H_5$ | $4-Cl-C_6H_4-CH_2$ | Cl | 164-166 |
| 103 | 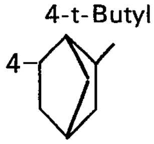 | $-CH_3$ | $-CH_3$ | $4-Cl-C_6H_4-CH_2$ | Cl | 234-236 |
| 104 | 4-Cyclohexyl | $-CH_3$ | $-CH_3$ | 4-tert.-Butyl$-C_6H_4-CH_2$ | Cl | 188-190 |
| 105 | 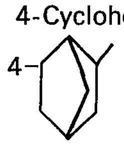 | $-CH_3$ | $-CH_3$ | 4-tert.-Butyl$-C_6H_4-CH_2$ | Cl | 208-210 |

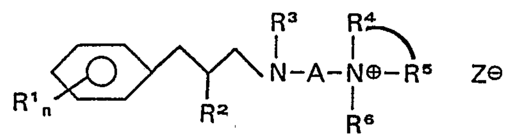

| Nr. | $R^1$ | $R^2$ | $R^3$ | A | $R^4 + R^5$ | $R^6$ | Z | Fp./°C |
|---|---|---|---|---|---|---|---|---|
| 84 | 4-$C(CH_3)_3$ | $-CH_3$ | $-CH_3$ | $-(CH_2)_2-$ | $-CH_2)_4-$ | $-CH_3$ | Br | 120 |
| 85 | 4-$C(CH_3)_3$ | $-CH_3$ | $-CH_3$ | $-(CH_2)_2-$ | $-(CH_2)_4-$ | Allyl | Br | Harz |
| 86 | 4-$C(CH_3)_3$ | $-CH_3$ | $-CH_3$ | $-(CH_2)_2-$ | $-(CH_2)_5-$ | Allyl | Br | Harz |
| 87 | 4-$C(CH_3)_3$ | $-CH_3$ | $-CH_3$ | $CH_3$<br>$-CH_2CH_2CH-$ | $-(CH_2)_5$ | Allyl | Br | Harz |
| 88 | 4-$C(CH_3)_3$ | $-CH_3$ | $-CH_3$ | $-(CH_2)_2-$ | $CH_3$<br>$-CH_2-CH_2-CH-CH_2-CH_2-$ | $C_6H_5-CH_2$ | Br | Harz |
| 89 | H | $-CH_3$ | $-CH_3$ | $-(CH_2)_2-$ | $CH_2-CH_2-O-CH_2-CH_2-$ | $-CH_3$ | Br | 185-188 |
| 90 | 4-$C(CH_3)_3$ | $-CH_3$ | $-CH_3$ | $-(CH_2)_2-$ | $CH_2-CH_2-O-CH_2-CH_2-$ | Buten-2-yl-1 | Br | 148-151 |
| 91 | H | $-CH_3$ | $-CH_3$ | $-(CH_2)_2-$ | $CH_2-CH_2-O-CH_2-CH_2-$ | $C_6H_5-CH_2-$ | Br | 125-128 |
| 92 | 4-$C(CH_3)_3$ | $-CH_3$ | $-CH_3$ | $-(CH_2)_2-$ | $CH_2-CH_2-O-CH_2-CH_2-$ | $C_6H_5-CH_2-$ | Br | 130-134 |
| 93 | 4-$C(CH_3)_3$ | $-CH_3$ | $-CH_3$ | $-(CH_2)_2-$ | $CH_3$ $CH_3$<br>$-CH_2-CH-O-CH-CH_2-$ | $C_6H_5-CH_2-$ | Br | Harz |
| 94 | 4-$C(CH_3)_3$ | $-CH_3$ | $-CH_3$ | $-(CH_2)_2-$ | $CH_3$ $CH_3$<br>$-CH_2-CH-O-CH-CH_2-$ | 4-$ClC_6H_4-CH_2-$ | Br | Harz |
| 95 | 4-$C(CH_3)_3$ | $-CH_3$ | $-CH_3$ | $-(CH_2)_2-$ | $CH_2-CH_2-O-CH_2-CH_2-$ | 4-$ClC_6H_4-CH_2-$ | Br | 126-133 |
| 96 | H | $-CH_3$ | $-CH_3$ | $-(CH_2)_2-$ | $CH_2-CH_2-O-CH_2-CH_2-$ | 4-(tert.-$C_4H_9)C_6H_4-CH_2-$ | Br | 145-148 |
| 97 | 4-$C(CH_3)_3$ | $-CH_3$ | $-CH_3$ | $-(CH_2)_2-$ | $CH_2-CH_2-O-CH_2-CH_2-$ | 4-(tert.-$C_4H_9)C_6H_4-CH_2-$ | Br | 120 (Zers.) |
| 98 | 4-$C(CH_3)_3$ | $-CH_3$ | $-CH_3$ | $-(CH_2)_2-$ | $CH_3$ $CH_3$<br>$-CH_2-CH-O-CH-CH_2-$ | 4-(tert.-$C_4H_9)C_6H_4-CH_2-$ | Br | Harz |

Ammoniakkomplex von
Zink-(N,N'-propylenbisdithiocarbamat) und
N,N'-Polypropylenbis(thiocarbamoyl)disulfid;
Nitroderivate, wie
Dinitro-(1-methylheptyl)phenylcrotonat,
2-sek.-Butyl-4,6-dinitrophenyl-3,3-dimethylacrylat,
2-sek.-Butyl-4,6-dinitrophenylisopropylcarbonat;
heterocyclische Substanzen, wie
N-(1,1,2,2-Tetrachlorethylthio)tetrahydrophthalimid,
N-Trichlormethylthiotetrahydrophthalimid,
2-Heptadecyl-2-imidazolinacetat,
2,4-Dichlor-6-(o-chloranilino)-s-triazin,
0,0-Dimethylphthalimidophosphonothioat,
5-Amino-1-(bis(dimethylamino)phosphinyl)-3-phenyl-1,2,4-triazol,
2,3-Dicyano-1,4-dithioanthrachinon,
2-Thio-1,3-dithio-4(4,5-b)-chinoxalin,
1-(Butylcarbamoyl)-2-benzimidazolcarbaminsäuremethylester,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-[Furyl-(2)]-benzimidazol,
Piperazin-1,4-diylbis-[1-(2,2,2-trichlorethyl)]-formamid,
2-[Thiazolyl-(4)]-benzimidazol,
5-Butyl-2-dimethylamino-4-hydroxy-6-methylpyrimidin,
Bis-(p-chlorphenyl)-3-pyridinmethanol,
1,2-Bis-(3-ethoxycarbonyl-2-thioureido)benzol,
1,2-Bis-(3-methoxycarbonyl-2-thioureido)benzol
sowie verschiedene Fungizide, wie
Dodecylguanidinacetat,
3-[3-(3,5-Dimethyl-2-oxycyclohexyl)-2-hydroxyethyl]glutarimid,
Hexachlorbenzol,
N-Dichlorfluormethylthio-N',N'-dimethyl-N-phenylschwefelsäurediamid,
2,5-Dimethylfuran-3-carbonsäureanilid,
2-Methylbenzoesäureanilid,
2-Jodbenzoesäureanilid,
1-(3,4-Dichloranilino)-1-formylamino-2,2,2-trichlorethan,
2,6-Dimethyl-N-tridecylmorpholin bzw. dessen Salze,
2,6-Dimethyl-N-cyclododecylmorpholin bzw. dessen Salze,
DL-Methyl-N-(2,6-dimethylphenyl)-N-furoyl-(2)-alaninat,
DL-N-(2,6-Dimethylphenyl)-N-(2'-methoxyacetyl)alaninmethylester,
5-Nitroisophthalsäurediisopropylester,
1-(1',2',4'-Triazolyl-1')-[1-(4'-chlorphenoxy)]-3,3-dimethylbutan-2-on,
1-(1',2',4'-Triazolyl-1')-[1-(4'-chlorphenoxy)]-3,3-dimethylbutan-2-ol,
N-(2,6-Dimethylphenyl)-N-chloracetyl-DL-2-aminobutyrolacton,
N-(n-Propyl)-N-(2,4,6-trichlorphenoxyethyl)-N'-imidazolylharnstoff,
N-Cyclohexyl-N-methoxy-2,5-dimethylfuran-3-carbonsäureamid,
2,4,5-Trimethylfuran-3-carbonsäureanilid,
5-Methyl-5-vinyl-3-(3,5-dichlorphenyl)-2,4-dioxo-1,3-oxazolidin,
5-Methoxymethyl-5-methyl-3-(3,5-dichlorphenyl)-2,4-dioxo-1,3-oxazolidin,
N-[3-(p-tert.-Butylphenyl)-2-methylpropyl]cis-2,6-dimethylmorpholin,
N-Formyl-N-morpholin-2,2,2-trichlorethylacetal,
1-[2-(2,4-Dichlorphenyl)-4-ethyl-1,3-dioxolan-2-ylmethyl]-[1H]-1,2,4-triazol,
1-[2-(2,4-Dichlorphenyl)-4-n-propyl-1,3-dioxolan-2-ylmethyl]-[1H]-1,2,4-triazol.

Die Wirkstoffe werden beispielsweise in Form von direkt versprühbaren Lösungen, Pulvern, Suspensionen, auch hochprozentige wässerige, ölige oder sonstige Suspensionen oder Dispersionen, Emulsionen, Öldispersionen, Pasten, Stäubemitteln, Streumitteln, Granulaten durch Versprühen, Vernebeln, Verstäuben, Verstreuen oder Giessen ausgebracht. Die Anwendungsformen richten sich ganz nach den Verwendungszwecken; sie sollten in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, Wasser usw., in Betracht.

Wässerige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuelle Lösungsmittel oder aus Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfate, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol-, Octylphenol-, Nonylphenol-, Alkylphenolpolyglykolether, Tributylphenylpolyglykolether, Alkylarylpolyetheralkohole, Isotridecylalkohol, Fettalkoholethylenoxidkondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kreide, Bolus, Löss, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nussschalenmehl, Cellulosepulver und andere feste Trägerstoffe.

Beispiele für solche Zubereitungen sind:

I. Man vermischt 90 Gew.-Teile der Verbindung 14 mit 10 Gew.-Teilen N-Methyl-α-pyrrolidon und erhält eine Lösung, die zur Anwendung in Form kleinster Tropfen geeignet ist.

II. 20 Gew.-Teile der Verbindung 15 werden in einer Mischung gelöst, die aus 80 Gew.-Teilen Xylol, 10 Gew.-Teilen des Anlagerungsproduktes von 8 bis 10 mol Ethylenoxid an 1 mol Ölsäure-N-monoethanolamid, 5 Gew.-Teilen Calciumsalz der Dodecylbenzolsulfonsäure und 5 Gew.-Teilen des Anlagerungsproduktes von 40 mol Ethylenoxid an 1 mol Rizinusöl besteht. Durch Ausgiessen und feines Verteilen der Lösung in Wasser erhält man eine wässerige Dispersion.

III. 20 Gew.-Teile der Verbindung 56 werden in einer Mischung gelöst, die aus 40 Gew.-Teilen Cyclohexanon, 30 Gew.-Teilen Isobutanol, 20 Gew.-Teilen des Anlagerungsproduktes von 40 mol Ethylenoxid an 1 mol Rizinusöl besteht. Durch Eingiessen und feines Verteilen der Lösung in Wasser erhält man eine wässerige Dispersion.

IV. 20 Gew.-Teile der Verbindung 57 werden in einer Mischung gelöst, die aus 25 Gew.-Teilen Cyclohexanol, 65 Gew.-Teilen einer Mineralölfraktion vom Sdp. 210° C und 10 Gew.-Teilen des Anlagerungsproduktes von 40 mol Ethylenoxid an 1 mol Rizinusöl besteht. Durch Eingiessen und feines Verteilen der Lösung in Wasser erhält man eine wässerige Dispersion.

V. 80 Gew.-Teile der Verbindung 63 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 5 Gew.-Teile der Verbindung 68 werden mit 95 Gew.-Teilen feinteiligem Kaolin innig vermischt. Man erhält auf diese Weise ein Stäubemittel, das 5 Gew.-% des Wirkstoffs enthält.

VII. 30 Gew.-Teile der Verbindung 69 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung 73 werden mit 30 Gew.-Teilen Natriumsalz eines Phenolsulfonsäure/Harnstoff/Formaldehyd-Kondensates, 2 Gew.-Teilen Kieselgel und 48 Gew.-Teilen Wasser innig vermischt. Man erhält eine stabile wässerige Dispersion.

IX. 20 Gew.-Teile der Verbindung 76 werden mit 2 Gew.-Teilen Calciumsalz der Dodecylbenzolsulfonsäure, 8 Gew.-Teilen Fettalkoholglykolether, 2 Gew.-Teilen Natriumsalz eines Phenolsulfonsäure/Harnstoff/Formaldehyd-Kondensates und 68 Gew.-Teilen eines paraffinischen Mineralöls innig vermischt. Man erhält eine stabile ölige Dispersion.

Die folgenden Versuche belegen die biologische Wirkung der neuen Verbindungen. Vergleichsmittel ist der bekannte Wirkstoff N-Trichlormethylthiotetrahydrophthalimid A.

*Versuch 1:*

*Wirksamkeit gegen* Botrytis cinerea

Paprikasämlige der Sorte Neusiedler Ideal Elite werden, nachdem sich 4 bis 5 Blätter gut entwikkelt haben, mit wässerigen Suspensionen, die 80% Wirkstoff und 20% Emulgiermittel in der Trockensubstanz enthalten, tropfnass gespritzt. Nach dem Antrocknen des Spritzbelages werden die Pflanzen mit einer Konidienaufschwemmung des Pilzes *Botrytis cinerea* besprüht und bei 22 bis 24° C in eine Kammer mit hoher Luftfeuchtigkeit gestellt. Nach 5 d hat sich die Krankheit auf den unbehandelten Kontrollpflanzen so stark entwickelt, dass die entstandenen Blattnekrosen den überwiegenden Teil der Blätter bedecken.

Das Ergebnis des Versuches zeigte, dass beispielsweise die Wirkstoffe 1, 2, 9, 12, 13, 14, 15, 16, 17, 18, 20, 21, 22, 23, 24, 25, 26, 27, 28, 29, 30, 31, 32, 33, 34, 35, 36, 37, 40, 46, 50, 58, 63, 64, 65, 66, 69, 70, 75, 78, 97, bei Anwendung als 0,05%ige Spritzbrühe, eine bessere Wirkung (beispielsweise 100%) hatten als der bekannte Wirkstoff A (beispielsweise 70%).

*Versuch 2:*

*Wirksamkeit gegen* Phytophthora infestans

Blätter von Topfplanzen der Sorte Grosse Fleischtomate werden mit wässeriger Spritzbrühe,

die 80% Wirkstoff und 20% Emulgiermittel in der Trockensubstanz enthält, besprüht. Nach dem Antrocknen des Spritzbelages werden die Blätter mit einer Zoosporenaufschwemmung des Pilzes *Phytophtora infestans* infiziert. Die Pflanzen werden dann in einer wasserdampfgesättigten Kammer bei Temperaturen zwischen 16 und 18° C aufgestellt. Nach 5 d hat sich die Krankheit auf den unbehandelten, jedoch infizierten Kontrollpflanzen so stark entwickelt, dass die fungizide Wirksamkeit der Substanzen beurteilt werden kann.

Das Ergebnis des Versuches zeigte, dass beispielsweise die Wirkstoffe 2, 31, 33, 34, 35, 40, 46, 57, 59, 60, 63, 65, 66, 68, 69, 73, 75, 78, bei Anwendung als 0,025%ige Spritzbrühe, eine bessere Wirkung (beispielsweise 97%) zeigten als der Wirkstoff A (beispielsweise 60%).

*Versuch 3:*

*Wirksamkeit gegen Rebenperonospora* (Plasmopara viticola)

Blätter von Topfreben der Sorte Müller-Thurgau werden mit wässeriger Spritzbrühe, die 80% Wirkstoff und 20% Emulgiermittel in der Trockensubstanz enthält, besprüht. Um die Wirkungsdauer der Wirkstoffe beurteilen zu können, werden die Pflanzen nach dem Antrocknen des Spritzbelages 10 d im Gewächshaus aufgestellt. Erst dann werden die Blätter mit einer Zoosporenaufschwemmung von *Plasmopara viticola* (Rebenperonospora) infiziert. Danach werden die Reben zunächst für 16 h in einer wasserdampfgesättigten Kammer bei 24° C und anschliessend für 8 d in einem Gewächshaus bei Temperaturen zwischen 20 und 30° C aufgestellt. Nach dieser Zeit werden die Pflanzen zur Beschleunigung des Sporangienträgerausbruches abermals für 16 h in der feuchten Kammer aufgestellt. Dann erfolgt die Beurteilung des Ausmasses des Pilzausbruches auf den Blattunterseiten.

Das Ergebnis des Versuches zeigte, dass beispielsweise die Wirkstoffe 40, 46, 59, 60, 63, 68, 69, 75, 78, bei Anwendung als 0,25%ige Spritzbrühe, eine gute fungizide Wirkung (beispielsweise 90%) zeigten.

*Versuch 4:*

*Wirksamkeit gegen* Aspergillus niger

Die Wirkstoffe werden einer für das Wachstum des Pilzes *Aspergillus niger* optimal geeigneten Nährlösung in Mengen von 100, 50, 25 und 10 Gew.-Teilen pro Million Teile Nährlösung zugesetzt. Es werden jeweils 20 ml der so behandelten Nährlösung in 100-ml-Glaskolben mit 0,3 mg *Aspergillus*-Pilzsporen beimpft. Die Kolben werden 120 h lang bei 36° C erwärmt und anschliessend das Ausmass der Pilzentwicklung, das bevorzugt auf der Nährlösungsoberfläche erfolgt, beurteilt.

Das Ergebnis des Versuches zeigte, dass beispielsweise die Wirkstoffe 1, 14, 15, 22, 28, 82, 83, beispielsweise bei Anwendung in Mengen von 100, 50, 25, 10 Gew.-Teilen Wirkstoff pro Million Teile Nährlösung eine gute Wirkung (beispielsweise 100%) zeigten.

*Versuch 5:*

*Wirksamkeit gegen* Staphylococcus aureus

Zur Ermittlung der Wirksamkeit der neuen Verbindungen gegenüber Bakterien werden zu je 5 ml steigender Verdünnungen der Wirkstoffe 5 ml doppelt konzentrierter Nährbouillon in sterile Reagenzgläser gegeben und vermischt. Durch Zugabe von einem Tropfen einer 1:10 verdünnten 16 h alten Bouillonkultur der Bakterienart *Staphylococcus aureus* werden dann die Röhrchen beimpft und 24 h bei 37° C bebrütet. Nach dieser Zeit werden Proben aus den Röhrchen auf Bakteriennährboden übertragen und diese ebenfalls 24 h lang bei 37° C bebrütet.

Das Ergebnis des Versuches zeigte, dass beispielsweise die Wirkstoffe 14, 15, 22, 25, 28, 32, 33, 34, 40, 41, 58, 59, 60, 63, 64, 66, 67, 70, 74, 77, 79, 81, 82, 83, 97, bei einer Verdünnung von 1:10000 (100 ppm), eine gute Wirkung (beispielsweise 100%) zeigten.

**Patentansprüche** für die Vertragsstaaten BE, CH, DE, FR, GB, IT, LI, NL, SE

1. Diaminderivat der Formel:

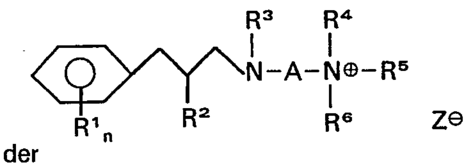

(I)

in der

$R^1$ Alkyl, halogensubstituiertes Alkyl, ggf. substituiertes Aryl oder Aralkyl, Cycloalkyl, Bicycloalkyl, Alkoxy, Acyl oder Halogen,

n 0, 1, 2 oder 3,

$R^2$ Alkyl, Alkenyl oder Alkoxy,

A eine ggf. durch Alkyl, Halogenalkyl, Alkoxy oder Halogen substituierte $C_2$-$C_4$-Kette oder ein ggf. durch Alkyl, Halogenalkyl oder Alkoxy substituiertes $C_5$-$C_7$-Cycloalkylen bedeutet,

$R^3$, $R^4$ und $R^5$ jeweils unabhängig voneinander Alkyl, halogensubstituiertes Alkyl, Alkenyl, Alkinyl oder ggf. durch Halogen, Alkyl, Alkoxy, Trifluormethyl, Cyan oder Nitro substituiertes Benzyl bedeuten, oder $R^3$ mit $R^4$ zusammen mit A und mit den beiden Stickstoffatomen, die an A gebunden sind, einen ggf. durch Alkyl substituierten Piperazin- oder Perhydrodiazepinring bilden oder $R^4$ zusammen mit $R^5$ und dem Stickstoffatom, dessen Substituenten sie sind, einen ggf. durch Alkyl substituierten 5-, 6- oder 7gliedrigen heterocyclischen Ring mit 1 bis 2 Heteroatomen bilden,

$R^6$ Alkyl, Alkenyl, Alkinyl oder ggf. substituiertes Aralkyl oder Aryloxyalkyl bedeutet, und

Z das Anion einer beliebigen, nicht phytotoxischen Säure bedeutet.

2. Diamin der Formel (I) gemäss Anspruch 1, dadurch gekennzeichnet, dass

$R^1$ Chlor, Brom, $C_1$-$C_8$-Alkyl, Cyclohexyl, Benzyl, Methoxy, Ethoxy oder Acetyl,

n 0, 1 oder 2,

$R^2$ $C_1$-$C_4$-Alkyl, $C_3$-$C_4$-Alkenyl, $C_1$-$C_3$-Alkoxy,

A $-(CH_2)_2-$, $-(CH_2)_3-$,
$CH_3$ $CH_3$ $CH_3$
$-CH-CH_2-$, $-CH_2-CH-$, $-CH-CH_2CH_2-$,

$R^3$ Methyl, Ethyl, n-Butyl, ggf. substituiertes Benzyl,

$R^4$ und $R^5$ unabhängig voneinander Methyl, Ethyl, Propyl, Butenyl und Propargyl oder $R^4$ mit $R^5$ zusammen mit dem N einen ggf. durch Methyl substituierten Pyrrolidin-, Piperidin- oder Morpholinring bilden,

$R^6$ $C_1$-$C_5$-Alklyl, Allyl, 2-Buten-1-yl, 4-Chlor-2-buten-1-yl, Propargyl, 1- oder 2-Naphthylmethyl, Benzyl, durch Fluor, Chlor, Iod, $C_1$-$C_5$-Alkyl, Methoxy, Trifluormethyl, Cyan oder Nitro substituiertes Benzyl oder ggf. durch Halogen, $C_1$-$C_4$-Alkyl, Methoxy oder Trifluormethyl substituiertes 1-Phenoxyethyl, 1-Phenoxypropyl oder 1-Phenoxybutyl oder den Rest 4-($C_1$-$C_8$-Alkyl)phenyl-(2-methyl-1-propyl), und

Z das Anion einer nicht phytotoxischen Säure bedeutet.

3. Fungizides Mittel, enthaltend ein Diaminderivat der Formel:

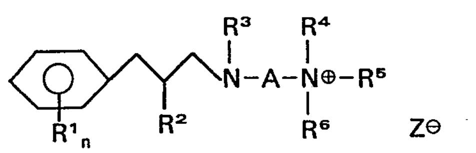
(I)

in der

$R^1$ Alkyl, halogensubstituiertes Alkyl, ggf. substituiertes Aryl oder Aralkyl, Cycloalkyl, Bicycloalkyl, Alkoxy, Acyl oder Halogen,

n 0, 1, 2 oder 3,

$R^2$ Alkyl, Alkenyl oder Alkoxy,

A eine ggf. durch Alkyl, Halogenalkyl, Alkoxy oder Halogen substituierte $C_2$-$C_4$-Kette oder ein ggf. durch Alkyl, Halogenalkyl oder Alkoxy substituiertes, $C_5$-$C_7$-Cycloalkylen bedeutet,

$R^3$, $R^4$ und $R^5$ jeweils unabhängig voneinander Alkyl, halogensubstituiertes Alkyl, Alkenyl, Alkinyl oder ggf. durch Halogen, Alkyl, Alkoxy, Trifluormethyl, Cyan oder Nitro substituiertes Benzyl bedeuten, oder $R^3$ mit $R^4$ zusammen mit A und mit den beiden Stickstoffatomen, die an A gebunden sind, einen ggf. durch Alkyl substituierten Piperazin- oder Perhydrodiazepinring bilden oder $R^4$ zusammen mit $R^5$ und dem Stickstoffatom, dessen Substituenten sie sind, einen ggf. durch Alkyl substituierten 5-, 6- oder 7gliedrigen heterocyclischen Ring mit 1 bis 2 Heteroatomen bilden,

$R^6$ Alkyl, Alkenyl, Alkinyl oder ggf. substituiertes Aralkyl oder Aryloxyalkyl bedeutet, und

Z das Anion einer beliebigen, nicht phytotoxischen Säure bedeutet.

4. Fungizides Mittel, enthaltend ein Diamin der Formel (I) gemäss Anspruch 1, dadurch gekennzeichnet, dass

$R^1$ Chlor, Brom, $C_1$-$C_8$-Alkyl, Cyclohexyl, Benzyl, Methoxy, Ethoxy oder Acetyl,

n 0, 1 oder 2,

$R^2$ $C_1$-$C_4$-Alkyl, $C_3$-$C_4$-Alkenyl, $C_1$-$C_3$-Alkoxy,

A $-(CH_2)_2-$, $-(CH_2)_3-$,
$CH_3$ $CH_3$ $CH_3$
$-CH-CH_2-$, $-CH_2-CH-$, $-CH-CH_2CH_2-$,

$R^3$ Methyl, Ethyl, n-Butyl, ggf. substituiertes Benzyl,

$R^4$ und $R^5$ unabhängig voneinander Methyl, Ethyl, Propyl, Butenyl und Propargyl oder $R^4$ mit $R^5$ zusammen mit dem N einen ggf. durch Methyl substituierten Pyrrolidin-, Piperidin- oder Morpholinring bilden.

$R^6$ $C_1$-$C_5$-Alkyl, Allyl, 2-Buten-1-yl, 4-Chlor-2-Buten-1-yl, Propargyl, 1- oder 2-Naphthylmethyl, Benzyl, durch Fluor, Chlor, Iod, $C_1$-$C_5$-Alkyl, Methoxy, Trifluormethyl, Cyan oder Nitro substituiertes Benzyl oder ggf. durch Halogen, $C_1$-$C_4$-Alkyl, Methoxy oder Trifluormethyl substituiertes 1-Phenoxyethyl, 1-Phenoxypropyl oder 1-Phenoxybutyl oder den Rest 4-($C_1$-$C_8$-Alkyl)-phenyl-(2-methyl-1-propyl), und

Z das Anion einer nicht phytotoxischen Säure bedeutet.

5. Bakterizides Mittel, enthaltend ein Diamin der Formel (I) gemäss Anspruch 1.

6. Verfahren zur Herstellung eines fungiziden Mittels, dadurch gekennzeichnet, dass man ein Diamin der Formel (I) gemäss Anspruch 1 mit einem festen oder flüssigen Trägerstoff mischt.

7. Verfahren zur Bekämpfung von Pilzen, dadurch gekennzeichnet, dass man ein Diamin der Formel (I) gemäss Anspruch 1 auf die Pilze oder auf durch Pilzbefall bedrohte Flächen, Materialien, Pflanzen oder Saatgüter einwirken lässt.

8. Verfahren zur Herstellung von Diaminen der Formel (I) gemäss Anspruch 1, dadurch gekennzeichnet, dass man ein Amin der Formel:

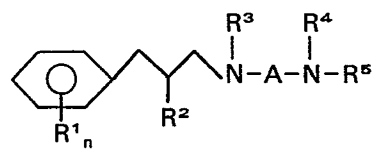
(II)

in der A, $R^1$, $R^2$, $R^3$, $R^4$, $R^5$ und n die in Anspruch 1 angegebenen Bedeutungen haben, mit einer Verbindung der Formel $R^6Z$, in der $R^6$ und Z die in Anspruch 1 angegebenen Bedeutungen haben, ggf. in Gegenwart eines Verdünnungsmittels umsetzt.

**Patentansprüche** für den Vertragsstaat: AT

1. Fungizides Mittel, enthaltend ein Diaminderivat der Formel:

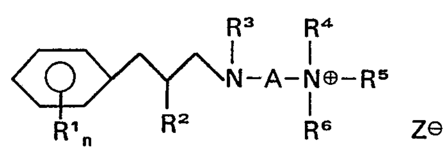
(I)

in der

$R^1$ Alkyl, halogensubstituiertes Alkyl, ggf. substituiertes Aryl oder Aralkyl, Cycloalkyl, Bicycloalkyl, Alkoxy, Acyl oder Halogen,

n 0, 1, 2 oder 3,

$R^2$ Alkyl, Alkenyl oder Alkoxy,

A eine ggf. durch Alkyl, Halogenalkyl, Alkoxy oder Halogen substituierte $C_2$-$C_4$-Kette oder ein ggf. durch Alkyl, Halogenalkyl oder Alkoxy substituiertes, $C_5$-$C_7$-Cycloalkylen bedeutet,

$R^3$, $R^4$ und $R^5$ jeweils unabhängig voneinander Alkyl, halogensubstituiertes Alkyl, Alkenyl, Alkinyl oder ggf. durch Halogen, Alkyl, Alkoxy, Trifluormethyl, Cyan oder Nitro substituiertes Benzyl bedeuten, oder $R^3$ mit $R^4$ zusammen mit A und mit den beiden Stickstoffatomen, die an A gebunden sind, einen ggf. durch Alkyl substituierten Piperazin- oder Perhydrodiazepinring bilden oder $R^4$ zusammen mit $R^5$ und dem Stickstoffatom, dessen Substituenten sie sind, einen ggf. durch Alkyl substituierten 5-, 6- oder 7gliedrigen heterocyclischen Ring mit 1 bis 2 Heteroatomen bilden,

$R^6$ Alkyl, Alkenyl, Alkinyl oder ggf. substituiertes Aralkyl oder Aryloxyalkyl bedeutet, und

Z das Anion einer beliebigen, nicht phytotoxischen Säure bedeutet.

2. Fungizides Mittel, enthaltend ein Diamin der Formel (I) gemäss Anspruch 1, dadurch gekennzeichnet, dass

$R^1$ Chlor, Brom, $C_1$-$C_8$-Alkyl, Cyclohexyl, Benzyl, Methoxy, Ethoxy oder Acetyl,

n 0, 1 oder 2,

$R^2$ $C_1$-$C_4$-Alkyl, $C_3$-$C_4$-Alkenyl, $C_1$-$C_3$-Alkoxy,

A $-(CH_2)_2-$, $-(CH_2)_3-$,

$-CH(CH_3)-CH_2-$, $-CH_2-CH(CH_3)-$, $-CH(CH_3)-CH_2CH_2-$,

$R^3$ Methyl, Ethyl, n-Butyl, ggf. substituertes Benzyl,

$R^4$ und $R^5$ unabhängig voneinander Methyl, Ethyl, Propyl, Butenyl und Propargyl oder $R^4$ mit $R^5$ zusammen mit dem N einen ggf. durch Methyl substituierten Pyrrolidin-, Piperidin- oder Morpholinring bilden.

$R^6$ $C_1$-$C_5$-Alkyl, Allyl, 2-Buten-1-yl, 4-Chlor-2-buten-1-yl, Propargyl, 1- oder 2-Naphthylmethyl, Benzyl, durch Fluor, Chlor, Iod, $C_1$-$C_5$-Alkyl, Methoxy, Trifluormethyl, Cyan oder Nitro substituiertes Benzyl oder ggf. durch Halogen, $C_1$-$C_4$-Alkyl, Methoxy oder Trifluormethyl substituiertes 1-Phenoxyethyl, 1-Phenoxypropyl oder 1-Phenoxybutyl oder den Rest 4-($C_1$-$C_8$-Alkyl)phenyl-(2-methyl-1-propyl), und

Z das Anion einer nicht phytotoxischen Säure bedeutet.

3. Bakterizides Mittel, enthaltend ein Diamin der Formel (I) gemäss Anspruch 1.

4. Verfahren zur Herstellung eines fungiziden Mittels, dadurch gekennzeichnet, dass man ein Diamin der Formel (I) gemäss Anspruch 1 mit einem festen oder flüssigen Trägerstoff mischt.

5. Verfahren zur Bekämpfung von Pilzen, dadurch gekennzeichnet, dass man ein Diamin der Formel (I) gemäss Anspruch 1 auf die Pilze oder auf durch Pilzbefall bedrohte Flächen, Materialien, Pflanzen oder Saatgüter einwirken lässt.

6. Verfahren zur Herstellung von Diaminen der Formel (I) gemäss Anspruch 1, dadurch gekennzeichnet, dass man ein Amin der Formel

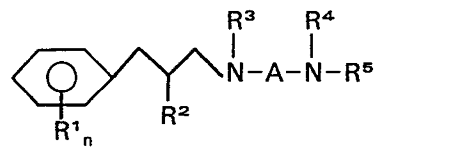

(II)

in der A, $R^1$, $R^2$, $R^3$, $R^4$, $R^5$ und n die in Anspruch 1 angegebenen Bedeutungen haben, mit einer Verbindung der Formel $R^6Z$, in der $R^6$ und Z die in Anspruch 1 angegebenen Bedeutungen haben, ggf. in Gegenwart eines Verdünnungsmittels umsetzt.

**Claims** for the Contracting States BE, CH, DE, FR, GB, IT, LI, NL, SE

1. A diamin derivative of the formula:

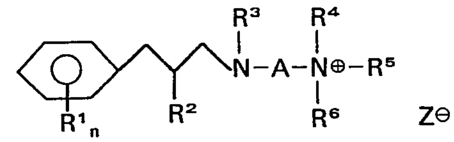

(I)

where

$R^1$ is unsubstituted or halogen-substituted alkyl, unsubstituted or substituted aryl or aralkyl, cycloalkyl, bicycloalkyl, alkoxy, acyl or halogen,

n is 0, 1, 2 or 3,

$R^2$ is alkyl, alkenyl or alkoxy,

A is a $C_2$-$C_4$-chain which is unsubstituted or substituted by alkyl, haloalkyl, alkoxy or halogen, or is $C_5$-$C_7$-cycloalkylene which is unsubstituted or substituted by alkyl, haloalkyl or alkoxy,

$R^3$, $R^4$ and $R^5$ independently of one another are unsubstituted or halogen-substituted alkyl, alkenyl, alkynyl, or benzyl which is unsubstituted or substituted by halogen, alkyl, alkoxy, trifluormethyl, cyano or nitro, or $R^3$ and $R^4$, together with A and the two nitrogen atoms which are bonded to A, form an unsubstituted or alkyl-substituted piperazine or perhydrodiazepine ring, or $R^4$ and $R^5$, together with the nitrogen atom of which they are substituents, form an unsubstituted or alkyl-substituted 5-membered, 6-membered or 7-membered heterocyclic ring containing 1 or 2 hetero atoms,

$R^6$ is alkyl, alkenyl, alkynyl or unsubstituted or substituted aralkyl or aryloxyalkyl, and

Z is an anion of any non-phytotoxic acid.

2. A diamine of the formula (I) as claimed in Claim 1, where

$R^1$ is chlorine, bromine, $C_1$-$C_8$-alkyl, cyclohexyl, benzyl, methoxy, ethoxy or acetyl,

n is 0, 1, or 2,

$R^2$ is $C_1$-$C_4$-alkyl, $C_3$-$C_4$-alkenyl, $C_1$-$C_3$-alkoxy,

A is $-(CH_2)_2-$, $-(CH_2)_3-$,

$-CH(CH_3)-CH_2-$, $-CH_2-CH(CH_3)-$, $-CH(CH_3)-CH_2CH_2-$,

$R^3$ is methyl, ethyl, n-butyl, or unsubstituted or substituted benzyl,

$R^4$ and $R^5$ independently of one another are methyl, ethyl, propyl, butenyl or propargyl, or $R^4$ and $R^5$, together with the nitrogen, are an

unsubstituted or methyl-substituted pyrrolidine, piperidine or morpholine ring,

$R^6$ is $C_1$-$C_5$-alkyl, allyl, but-2-en-1-yl, 4-chlorobut-2-en-1-yl, propargyl, naphth-1-ylmethyl, naphth-2-ylmethyl, unsubstituted benzyl, benzyl substituted by fluorine, chlorine, iodine, $C_1$-$C_5$-alkyl, methoxy, trifluoromethyl, cyano or nitro, $R^6$ is further 1-phenoxyethyl, 1-phenoxypropyl or 1-phenoxybutyl, each of which is unsubstituted or substituted by halogen, $C_1$-$C_4$-alkyl, methoxy or trifluoromethyl, or $R^6$ is 4-($C_1$-$C_8$-alkyl)phenyl-(2-methyl-1-propyl), and

Z is an anion of a non-phytotoxic acid.

3. A fungicidal agent containing a diamine of the formula:

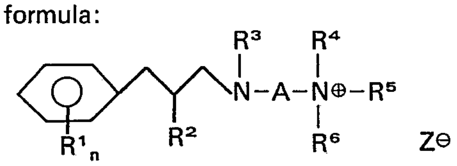

(I)

where

$R^1$ is unsubstituted or halogen-substituted alkyl, unsubstituted or substituted aryl or aralkyl, cycloalkyl, bicycloalkyl, alkoxy, acyl or halogen,

n is 0, 1, 2 or 3,

$R^1$ is alkyl, alkenyl or alkoxy,

A is a $C_2$-$C_4$-chain which is unsubstituted or substituted by alkyl, haloalkyl, alkoxy or halogen, or is $C_5$-$C_7$-cycloalkylene which is unsubstituted or substituted by alkyl, haloalkyl or alkoxy,

$R^3$, $R^4$ and $R^5$ independently of one another are unsubstituted or halogen-substituted alkyl, alkenyl, alkynyl, or benzyl which is unsubstituted or substituted by halogen, alkyl, alkoxy, trifluoromethyl, cyano or nitro, or $R^3$ and $R^4$, together with A and the two nitrogen atoms which are bonded to A, form an unsubstituted or alkyl-substituted piperazine or perhydrodiazepine ring, or $R^4$ and $R^5$, together with the nitrogen atom of which they are substituents, form an unsubstituted or alkyl-substituted 5-membered, 6-membered or 7-membered heterocyclic ring containing 1 or 2 hetero atoms,

$R^6$ is alkyl, alkenyl, alkynyl or unsubstituted or substituted aralkyl or aryloxyalkyl, and

Z is an anion of any non-phytotoxic acid.

4. A fungicidal agent containing a diamine of the formula (I) as claimed in Claim 1, where

$R^1$ is chlorine, bromine, $C_1$-$C_8$-alkyl, cyclohexyl, benzyl, methoxy, ethoxy or acetyl,

n is 0, 1 or 2,

$R^2$ is $C_1$-$C_4$-alkyl, $C_3$-$C_4$-alkenyl, $C_1$-$C_3$-alkoxy,

A is $-(CH_2)_2-$, $-(CH_2)_3-$, $-CH(CH_3)-CH_2-$, $-CH_2-CH(CH_3)-$, $-CH(CH_3)-CH_2CH_2-$,

$R^3$ is methyl, ethyl, n-butyl, or unsubstituted or substituted benzyl,

$R^4$ and $R^5$ independently of one another are methyl, ethyl, propyl, butenyl or propargyl, or $R^4$ and $R^5$, together with the nitrogen, are an unsubstituted or methyl-substituted pyrrolidine, piperidine or morpholine ring,

$R^6$ is $C_1$-$C_6$-alkyl, allyl, but-2-en-1-yl, 4-chlorobut-2-en-1-yl, propargyl, naphth-1-ylmethyl, naphth-2-ylmethyl, unsubstituted benzyl, benzyl substituted by fluorine, chlorine, iodine, $C_1$-$C_5$-alkyl, methoxy, trifluoromethyl, cyano or nitro, $R^6$ is further 1-phenoxyethyl, 1-phenoxypropyl or 1-phenoxybutyl, each of which is unsubstituted or substituted by halogen, $C_1$-$C_4$-alkyl, methoxy or trifluoromethyl, or $R^6$ is 4-($C_1$-$C_8$-alkyl)phenyl-(2-methyl-1-propyl), and

Z is an anion of a non-phytotoxic acid.

5. A bactericidal agent containing a diamine of the formula (I) as claimed in Claim 1.

6. A process for producing a fungicidal agent, wherein a diamine of the formula (I) as claimed in Claim 1 is mixed with a solid or liquid carrier.

7. A process for combating fungi, wherein a diamine of the formula (I) as claimed in Claim 1 is allowed to act on the fungi or the areas, materials, plants or seed threatened by fungus attack.

8. A process for preparing a diamine of the formula (I) as claimed in Claim 1, wherein an amine of the formula:

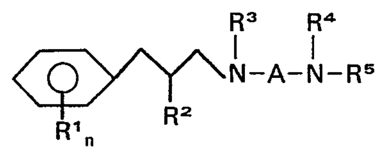

(II)

where A, $R^1$, $R^2$, $R^3$, $R^4$, $R^5$ and n have the meanings given in Claim 1, is reacted with a compound of the formula $R^6Z$, where $R^6$ and Z have the meanings given in Claim 1, in the presence or absence of a diluent.

**Claims** for the Contracting State: AT

1. A fungicidal agent containing a diamine derivative of the formula:

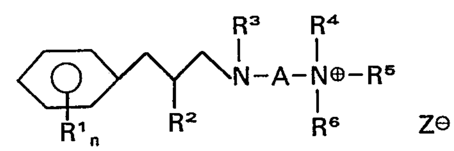

(I)

where $R^1$ is unsubstituted or halogen-substituted alkyl, unsubstituted or substituted aryl or aralkyl, cycloalkyl, bicycloalkyl, alkoxy, acyl or halogen, n is 0, 1, 2 or 3, $R^2$ is alkyl, alkenyl or alkoxy, A is a $C_2$-$C_4$-chain which is unsubstituted or substituted by alkyl, haloalkyl, alkoxy or halogen, or is $C_5$-$C_7$-cycloalkylene which is unsubstituted or substituted by alkyl, haloalkyl or alkoxy, $R^3$, $R^4$ and $R^5$ independently of one another are unsubstituted or halogen-substituted alkyl, alkenyl, alkynyl, or benzyl which is unsubstituted or substituted by halogen, alkyl, alkoxy, trifluoromethyl, cyano or nitro, or $R^3$ and $R^4$, together with A and the two nitrogen atoms which are bonded to A, form an unsubstituted or alkyl substituted piperazine or perhydrodiazepine ring, or $R^4$ and $R^5$, together with the nitrogen atom of which they are substituents, form an unsubstituted

or alkyl-substituted 5-membered, 6-membered or 7-membered heterocyclic ring containing 1 or 2 hetero atoms, $R^6$ is alkyl, alkenyl, alkynyl or unsubstituted or substituted aralkyl or aryloxyalkyl, and Z is an anion of any non-phytotoxic acid.

2. A fungicidal agent containing a diamine of the formula (I) as defined in Claim 1, where $R^1$ is chlorine, bromine, $C_1$-$C_8$-alkyl, cyclohexyl, benzyl, methoxy, ethoxy or acetyl, n is 0, 1 or 2, $R^2$ is $C_1$-$C_4$-alkyl, $C_3$-$C_4$-alkenyl, $C_1$-$C_3$-alkoxy,
A is $-(CH_2)_2-$, $-(CH_2)_3-$,
$-CH(CH_3)-CH_2-$, $-CH_2-CH(CH_3)-$, $-CH(CH_3)-CH_2CH_2-$,
$R^3$ is methyl, ethyl, n-butyl, or unsubstituted or substituted benzyl, $R^4$ and $R^5$ independently of one another are methyl, ethyl, propyl, butenyl or propargyl, or $R^4$ and $R^5$, together with the nitrogen, are an unsubstituted or methyl-substituted pyrrolidine, piperidine or morpholine ring, $R^6$ is $C_1$-$C_5$-alkyl, allyl, but-2-en-1-yl, 4-chlorobut-2-en-1-yl, propargyl, naphth-1-ylmethyl, naphth-2-ylmethyl, unsubstituted benzyl, benzyl substituted by fluorine, chlorine, iodine, $C_1$-$C_5$-alkyl, methoxy, trifluoromethyl, cyano or nitro, $R_6$ is further 1-phenoxyethyl, 1-phenoxypropyl or 1-phenoxybutyl, each of which is unsubstituted or substituted by halogen, $C_1$-$C_4$-alkyl, methoxy or trifluoromethyl, or $R^6$ is 4-($C_1$-$C_8$-alkyl)phenyl-(2-methyl-1-propyl), and Z is an anion of a non-phytotoxic acid.

3. A bactericidal agent containing a diamine of the formula (I) as defined in Claim 1.

4. A process for producing a fungicidal agent, wherein a diamine of the formula (I) as defined in Claim 1 is mixed with a solid or liquid carrier.

5. A process for combating fungi, wherein a diamine of the formula (I) as defined in Claim 1 is allowed to act on the fungi or the areas, materials, plants or seed threatened by fungus attack.

6. A process for preparing a diamine of the formula (I) as defined in Claim 1, wherein an amine of the formula:

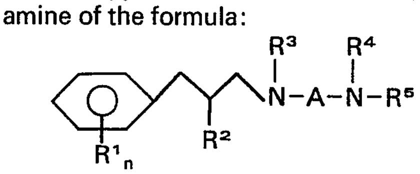 (II)

where A, $R^1$, $R^2$, $R^3$, $R^4$, $R^5$ and n have the meanings given in Claim 1, is reacted with a compound of the formula $R^6Z$, where $R^6$ and Z have the meanings given in Claim 1, in the presence or absence of a diluent.

**Revendications** pour les Etats contractants BE, CH, DE, FR, GB, IT, LI, NL, SE

1. Dérivé de diamine de formule:

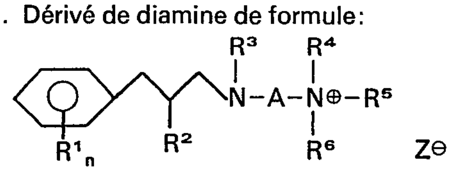 (I)

dans laquelle:
$R^1$ représente alkyle, alkyle halogénosubstitué, aryle ou aralkyle éventuellement substitué, cycloalkyle, bicycloalkyle, alcoxy, acyle ou halogène,
n représente 0, 1, 2 ou 3,
$R^2$ représente alkyle, alcényle ou alcoxy,
A représente une chaîne en $C_2$-$C_4$ éventuellement substituée par alkyle, halogénoalkyle, alcoxy ou halogène, ou un cycloalkylène en $C_5$-$C_7$ éventuellement substitué par alkyle, halogénoalkyle ou alcoxy,
$R^3$, $R^4$ et $R^5$ représentent chacun, indépendamment l'un de l'autre, alkyle, alkyle halogénosubstitué, alcényle, alcinyle ou benzyle éventuellement substitué par halogène, alkyle, alcoxy, trifluorométhyle, cyano ou nitro, ou $R^3$ et $R^4$ ensemble, avec A et avec les deux atomes d'azote qui sont reliés à A, forment un noyau pipérazine ou perhydrodiazépine éventuellement substitué par alkyle, ou $R^4$ ensemble avec $R^5$ et l'atome d'azote, dont ils sont les substituants, forment un noyau hétérocyclique de 5, 6 ou 7 chaînons, avec 1 ou 2 hétéroatomes, éventuellement substitué par alkyle,
$R^6$ représente alkyle, alcényle, alcinyle ou aralkyle ou aryloxyalkyle éventuellement substitué, et
Z représente l'anion d'un acide quelconque, non phytotoxique.

2. Diamine de formule (I) selon la revendication 1, caractérisée par le fait que:
$R^1$ représente chlore, brome, alkyle en $C_1$-$C_8$, cyclohexyle, benzyle, méthoxy, éthoxy ou acétyle,
n représente 0, 1 ou 2,
$R^2$ représente alkyle en $C_1$-$C_4$, alcényle en $C_3$-$C_4$, alcoxy en $C_1$-$C_3$,
A représente $-(CH_2)_2-$, $-(CH_2)_3-$,
$-CH(CH_3)-CH_2-$, $-CH_2-CH(CH_3)-$, $-CH(CH_3)-CH_2CH_2-$,
$R^3$ représente méthyle, éthyle, n-butyle, benzyle éventuellement substitué,
$R^4$ et $R^5$ représentent, indépendamment l'un de l'autre, méthyle, éthyle, propyle, butényle et propargyle, ou $R^4$ et $R^5$ ensemble, avec le N, forment un noyau pyrrolidine, pipéridine ou morpholine, éventuellement substitué par méthyle,
$R^6$ représente alkyle en $C_1$-$C_5$, allyle, 2-butén-1-yle, 4-chloro-2-butén-1-yle, propargyle, 1- ou 2-naphtylméthyle, benzyle, benzyle substitué par fluor, chlore, iode, alkyle en $C_1$-$C_5$, méthoxy, trifluorométhyle, cyano ou nitro, ou 1-phénoxyéthyle, 1-phénoxypropyle ou 1-phénoxybutyle éventuellement substitué par halogène, alkyle en $C_1$-$C_4$, méthoxy ou trifluorométhyle, ou le reste 4-(alkyle en $C_1$-$C_8$)-phényl-(2-méthyl-1-propyle), et
Z représente l'anion d'un acide non phytotoxique.

3. Agent fongicide contenant un dérivé de diamine de formule:

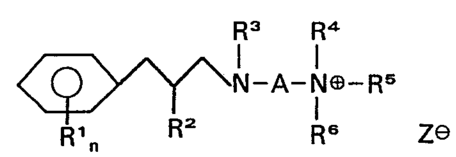 (I)

dans laquelle:

$R^1$ représente alkyle, alkyle halogénosubstitué, aryle ou aralkyle éventuellement substitué, cycloalkyle, bicycloalkyle, alcoxy, acyle ou halogène,

n représente 0, 1, 2 ou 3,

$R^2$ représente alkyle, alcényle ou alcoxy,

A représente une chaîne en $C_2$-$C_4$ éventuellement substituée par alkyle, halogénoalkyle, alcoxy ou halogène, ou un cycloalkylène en $C_5$-$C_7$ éventuellement substitué par alkyle, halogénoalkyle ou alcoxy,

$R^3$, $R^4$ et $R^5$ représentent chacun, indépendamment l'un de l'autre, alkyle, alkyle halogénosubstitué, alcényle, alcinyle ou benzyle éventuellement substitué par halogène, alkyle, alcoxy, trifluorométhyle, cyano ou nitro, ou $R^3$ et $R^4$ ensemble, avec A et avec les deux atomes d'azote qui sont reliés à A, forment un noyau pipérazine ou perhydrodiazépine éventuellement substitué par alkyle, ou $R^4$ ensemble avec $R^5$ et l'atome d'azote, dont ils sont les substituants, forment un noyau hétérocyclique de 5, 6 ou 7 chaînons, avec 1 ou 2 hétéroatomes, éventuellement substitué par alkyle,

$R^6$ représente alkyle, alcényle, alcinyle ou aralkyle ou aryloxyalkyle éventuellement substitué, et

Z représente l'anion d'un acide quelconque, non phytotoxique.

4. Agent fongicide contenant une diamine de formule (I) selon la revendication 1, caractérisée par le fait que

$R^1$ représente chlore, brome, alkyle en $C_1$-$C_8$, cyclohexyle, benzyle, méthoxy, éthoxy ou acétyle,

n représente 0, 1 ou 2,

$R^2$ représente alkyle en $C_1$-$C_4$, alcényle en $C_3$-$C_4$, alcoxy en $C_1$-$C_3$,

A représente $-(CH_2)_2-$, $-(CH_2)_3$,

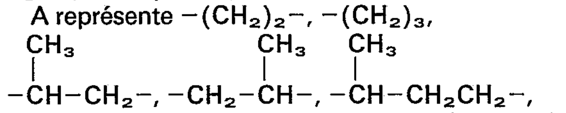

$R^3$ représente méthyle, éthyle, n-butyle, benzyle éventuellement substitué,

$R^4$ et $R^5$ représentent, indépendamment l'un de l'autre, méthyle, éthyle, propyle, butényle et propargyle, ou $R^4$ et $R^5$ ensemble, avec le N, forment un noyau pyrrolidine, pipéridine ou morpholine, éventuellement substitué par méthyle,

$R^6$ représente alkyle en $C_1$-$C_5$, allyle, 2-butén-1-yle, 4-chloro-2-butén-1-yle, propargyle, 1- ou 2-naphtylméthyle, benzyle, benzyle substitué par fluor, chlore, iode, alkyle en $C_1$-$C_5$, méthoxy, trifluorométhyle, cyano ou nitro, ou 1-phénoxyéthyle, 1-phénoxypropyle ou 1-phénoxybutyle éventuellement substitué par halogène, alkyle en $C_1$-$C_4$, méthoxy ou trifluorométhyle, ou le reste 4-(alkyle en $C_1$-$C_8$)-phényle-(2-méthyl-1-propyle), et

Z représente l'anion d'un acide non phytotoxique.

5. Agent bactéricide contenant une diamine de formule (I) selon la revendication 1.

6. Procédé de préparation d'un agent fongicide, caractérisé par le fait qu'on mélange une diamine de formule (I) selon la revendication 1 avec un support solide ou liquide.

7. Procédé de lutte contre les champignons, caractérisé par le fait qu'on fait agir une diamine de formule (I) selon la revendication 1 sur les champignons ou sur les surfaces, matériaux, plantes ou semences menacés par une maladie cryptogamique.

8. Procédé de préparation de diamine de formule (I) selon la revendication 1, caractérisé par le fait qu'on fait réagir une amine de formule:

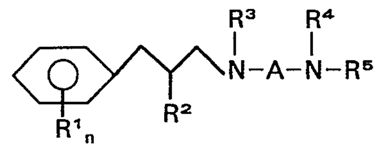

(II)

dans laquelle A, $R^1$, $R^2$, $R^3$, $R^4$, $R^5$ et n ont les significations indiquées dans la revendication 1, avec un composé de formule $R^6Z$, où $R^6$ et Z ont les significations données dans la revendication 1, éventuellement en présence d'un diluant.

**Revendications** pour l'Etat contractant: AT

1. Agent fongicide, contenant un dérivé de diamine de formule:

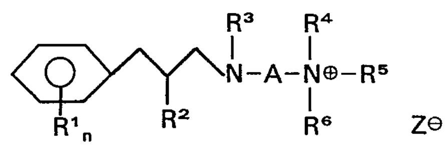

(I)

dans laquelle:

$R^1$ représente alkyle, alkyle halogénosubstitué, aryle ou aralkyle éventuellement substitué, cycloalkyle, bicycloalkyle, alcoxy, acyle ou halogène,

n représente 0, 1, 2 ou 3,

$R^2$ représente alkyle, alcényle ou alcoxy,

A représente une chaîne en $C_2$-$C_4$ éventuellement substituée par alkyle, halogénoalkyle, alcoxy ou halogène, ou un cycloalkylène en $C_5$-$C_7$ éventuellement substitué par alkyle, halogénoalkyle ou alcoxy,

$R^3$, $R^4$ et $R^5$ représentent chacun, indépendamment l'un de l'autre, alkyle, alkyle halogénosubstitué, alcényle, alcinyle ou benzyle éventuellement substitué par halogène, alkyle, alcoxy, trifluorométhyle, cyano ou nitro, ou $R^3$ et $R^4$ ensemble, avec A et avec les deux atomes d'azote qui sont reliés à A, forment un noyau pipérazine ou perhydrodiazépine éventuellement substitué par alkyle, ou $R^4$ ensemble avec $R^5$ et l'atome d'azote, dont ils sont les substituants, forment un noyau hétérocyclique de 5, 6 ou 7 chaînons, avec 1 ou 2 hétéroatomes, éventuellement substitué par alkyle,

$R^6$ représente alkyle, alcényle, alcinyle ou aralkyle ou aryloxyalkyle éventuellement substitué, et

Z représente l'anion d'un acide quelconque, non phytotoxique.

2. Agent fongicide contenant une diamine de

formule (I) selon la revendication 1, caractérisé par le fait que:

$R^1$ représente chlore, brome, alkyle en $C_1$-$C_8$, cyclohexyle, benzyle, méthoxy, éthoxy ou acétyle,

n représente 0, 1 ou 2,

$R^2$ représente alkyle en $C_1$-$C_4$, alcényle en $C_3$-$C_4$, alcoxy en $C_1$-$C_3$,

A représente $-(CH_2)_2-$, $-(CH_2)_3-$, $-CH(CH_3)-CH_2-$, $-CH_2-CH(CH_3)-$, $-CH(CH_3)-CH_2CH_2-$,

$R^3$ représente éthyle, n-butyle, benzyle éventuellement substitué,

$R^4$ et $R^5$ représentent, indépendamment l'un de l'autre, méthyle, éthyle, propyle, butényle et propargyle, ou $R^4$ et $R^5$ ensemble, avec le N, forment un noyau pyrrolidine, pipéridine ou morpholine, éventuellement substitué par méthyle,

$R^6$ représente alkyle en $C_1$-$C_5$, allyle, 2-butén-1-yle, 4-chloro-2-butén-1-yle, propargyle, 1- ou 2-naphtylméthyle, benzyle, benzyle substitué par fluor, chlore, iode, alkyle en $C_1$-$C_5$, méthoxy, trifluorométhyle, cyano ou nitro, ou 1-phénoxyéthyle, 1-phénoxypropyle, ou 1-phénoxybutyle éventuellement substitué par halogène, alkyle en $C_1$-$C_4$, méthoxy ou trifluorométhyle, ou le reste 4-(alkyle en $C_1$-$C_8$)-phényl-(2-méthyl-1-propyle), et

Z représente l'anion d'un acide phytotoxique.

3. Agent bactéricide contenant une diamine de formule (I) selon la revendication 1.

4. Procédé de préparation d'un agent fongicide, caractérisé par le fait qu'on mélange une diamine de formule (I) selon la revendication 1 avec un support solide ou liquide.

5. Procédé de lutte contre les champignons, caractérisé par le fait qu'on fait agir une diamine de formule (I) selon la revendication 1 sur les champignons ou sur les surfaces, matériaux, plantes ou semences menacés par une maladie cryptogamique.

6. Procédé de préparation de diamines de formule (I) selon la revendication 1, caractérisé par le fait qu'on fait réagir une amine de formule:

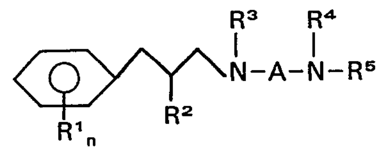

(II)

dans laquelle A, $R^1$, $R^2$, $R^3$, $R^4$, $R^5$ et n ont les significations indiquées dans la revendication 1, avec un composé de formule $R^6Z$, où $R^6$ et Z ont les significations données dans la revendication 1, éventuellement en présence d'un diluant.